(19) Europäisches Patentamt
European Patent Office
Office européen des brevets

(11) Veröffentlichungsnummer: **0 082 113**
**A1**

(12) **EUROPÄISCHE PATENTANMELDUNG**

(21) Anmeldenummer: **82810511.4**

(22) Anmeldetag: **29.11.82**

(51) Int. Cl.³: **C 07 D 499/00**, A 61 K 31/43
// C07D515/04, C07D205/08, C07F9/65, C07F7/18

(30) Priorität: **04.12.81 US 327380**
**06.05.82 CH 2805/82**

(43) Veröffentlichungstag der Anmeldung: **22.06.83**
**Patentblatt 83/25**

(84) Benannte Vertragsstaaten: **AT BE CH DE FR IT LI LU NL SE**

(71) Anmelder: **CIBA-GEIGY AG, Patentabteilung Postfach, CH-4002 Basel (CH)**

(72) Erfinder: **Pfaendler, Hans-Rudolf, Dr., Pippinplatz, D-8000 München 71 (DE)**
Erfinder: **Schneider, Peter, Dr., Rheinfelderstrasse 21A, CH-4058 Basel (CH)**

(54) **2-Aminobutyl-2-penem Derivate, Verfahren zu ihrer Herstellung und sie enthaltende pharmazeutische Präparate.**

(57) 2-Aminobutyl-6-hydroxymethyl-2-penem-3-carbonsäure-Verbindungen der Formel

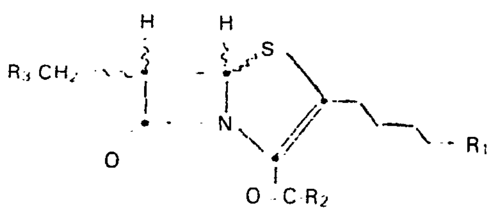

(I)

worin $R_1$ eine gegebenenfalls substituierte Aminogruppe darstellt, $R_2$ Hydroxy oder einen zusammen mit der Carbonylgruppe -C(=O) eine geschützte Carboxylgruppe bildenden Rest $R_2$ ^A bedeutet und $R_3$ eine gegebenenfalls geschützte Hydroxygruppe ist, ihre Salze, ihre optischen Isomeren und Mischungen von diesen optischen Isomeren besitzen antibiotische Eigenschaften. Ebenfalls umfasst sind Ausgangsstoffe.

EP 0 082 113 A1

CIBA-GEIGY AG 4-13909/+

Basel (Schweiz)

Aminobutyl-Verbindungen

Die Erfindung betrifft neue 2-Aminobutyl-6-hydroxymethyl-2-penem-verbindungen, Verfahren zu ihrer Herstellung und pharmazeutische Präparate, die solche Verbindungen enthalten.

Aus der Deutschen Offenlegungsschrift 29 50 898 und aus der Europäischen Patentanmeldung Nr. 3960 sind 2-Penem-Verbindungen bekannt geworden, die in 2-Stellung einen Aminoniederalkyl-Substituenten und in 6-Stellung eine 1-Hydroxyniederalkylgruppe enthalten. Diese Verbindungen sind wertvolle, antibiotisch wirksame Substanzen, die insbesondere als antibakterielle Antibiotika verwendet werden können. Als besonders bevorzugt werden in den genannten Schriften solche Penem-Verbindungen herausgestellt, die in 6-Stellung eine 1-Hydroxyäthylgruppe enthalten und damit, zusätzlich zu den im Penemgerüst bereits vorhandenen zwei Asymmetriezentren (C-Atome 5 und 6) ein weiteres Asymmetriezentrum am C-Atom 1' der Seitenkette besitzen. Die Trennung der daraus resultierenden vier möglichen Enantiomerenpaare erweist sich in der Regel als kompliziert, aufwendig und damit kostspielig.

2-Aminobutyl-6-hydroxymethyl-2-penem-Verbindungen sind bisher noch nicht bekannt geworden. Es wurde die überraschende Feststellung gemacht, dass solche Verbindungen neben der parenteralen auch eine hohe orale Wirksamkeit aufweisen. Da sie nur die beiden, dem Penemsystem eigenen Asymmetriezentren aufweisen und daher auch nur zwei Enantiomerenpaare möglich sind, wird die Isolierung der pharmakologisch besonders aktiven Stereoisomeren im hohen Masse erleichtert.

BAD ORIGINAL

Die Erfindung betrifft insbesondere neue 2-Aminobutyl-6-hydroxymethyl-2-penem-Verbindungen der Formel

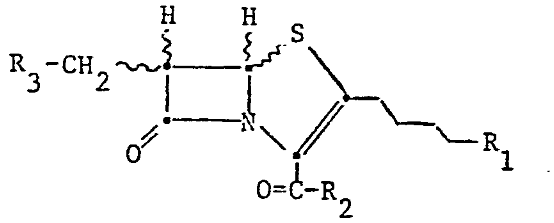

(I)

worin $R_1$ eine gegebenenfalls substituierte Aminogruppe darstellt, $R_2$ Hydroxy oder eine zusammen mit der Carbonylgruppe -C(=O)- eine geschützte Carboxylgruppe bildenden Rest $R_2^A$ bedeutet, und $R_3$ eine gegebenenfalls geschützte Hydroxygruppe ist, ihre Salze, ihre optischen Isomeren und Mischungen ihrer optischen Isomeren, Verfahren zu ihrer Herstellung, pharmazeutische Präparate enthaltend solche Verbindungen und ihre Verwendung als Antibiotika.

Die vor- und nachstehend verwendeten Definitionen haben im Rahmen der vorliegenden Beschreibung vorzugsweise die folgenden Bedeutungen:

Eine substituierte Aminogruppe $R_1$ ist eine geschützte Aminogruppe oder auch eine Methylenaminogruppe, worin der Methylenrest vorzugsweise mono- oder disubstituiert ist, z.B. eine Gruppe der Formel

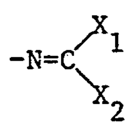

(IA),

worin $X_1$ Wasserstoff, gegebenenfalls substituiertes Amino, z.B. Amino, Niederalkylamino, Diniederalkylamino, Niederalkylenamino, Nitroamino, Hydrazino oder Anilino, veräthertes Hydroxy, z.B. Niederalkoxy oder Phenylniederalkoxy, veräthertes Mercapto, z.B. Niederalkylthio, gegebenenfalls substituiertes Niederalkyl, z.B. Niederalkyl, Aminoniederalkyl, N-Niederalkylaminoniederalkyl oder N,N-Diniederalkylaminoniederalkyl, Niederalkenyl, Phenyl oder monocyclisches Heteroaryl, wie entsprechendes 5- oder 6-gliedriges Heteroaryl mit 1 bis 2 Stick-

BAD ORIGINAL

Stickstoffatomen und/oder einem Sauerstoff- oder Schwefelatom, und $X_2$ gegebenenfalls substituiertes Amino, z.B. Amino, Niederalkylamino, Diniederalkylamino, Niederalkylenamino, Nitroamino, Hydrazino oder Anilino, veräthertes Hydroxy, z.B. Niederalkoxy oder Phenylniederalkoxy, oder veräthertes Mercapto, z.B. Niederalkylthio, darstellt.

In der vorliegenden Beschreibung bedeutet der im Zusammenhang mit Definitionen von Gruppen und Verbindungen verwendete Ausdruck "Nieder", dass die entsprechenden Gruppen bzw. Verbindungen, sofern nicht ausdrücklich anders definiert, bis zu 7, bevorzugt bis zu 4 Kohlenstoffatome enthalten.

Niederalkylamino ist z.B. Methylamino, Aethylamino, n-Propylamino, Isopropylamino oder n-Butylamino, während Diniederalkylamino z.B. Dimethylamino, Diäthylamino, Di-n-propylamino oder Di-n-butylamino bedeutet.

Niederalkylenamino weist insbesondere 4 bis 6 Kohlenstoffkettenglieder auf und bedeutet z.B. Pyrrolidino oder Piperidino.

Niederalkoxy ist z.B. Methoxy, Aethoxy, n-Propoxy, Isopropoxy, n-Butoxy oder tert.-Butoxy, während Phenylniederalkoxy z.B. Benzyloxy ist.

Niederalkylthio ist z.B. Methylthio, Aethylthio, n-Propylthio, Isopropylthio oder n-Butylthio.

Niederalkyl ist z.B. Methyl, Aethyl, n-Propyl, Isopropyl, n-Butyl, Isobutyl, sek.-Butyl oder tert.-Butyl.

Aminoniederalkyl ist z.B. 2-Aminoäthyl oder 3-Aminopropyl.

BAD ORIGINAL

N-Niederalkylaminoniederalkyl ist z.B. 2-Methyl- oder 2-Aethylaminoäthyl, während N,N-Diniederalkylaminoniederalkyl z.B. 2-Dimethylaminoäthyl oder 2-Diäthylaminoäthyl bedeutet.

Niederalkenyl ist z.B. Allyl,n-Propenyl oder Isopropenyl.

Monocyclisches 5- oder 6-gliedriges Heteroaryl mit 1 bis 2 Stickstoffatomen und/oder einem Sauerstoff- oder Schwefelatom ist z.B. Furyl, wie 2-Furyl, Thienyl, wie 2-Thienyl, Oxazolyl, wie 2-Oxazolyl, Thiazolyl, wie 2- oder 4-Thiazolyl, Pyridyl, wie 2-, 3- oder 4-Pyridyl, oder Pyrimidyl, wie 2-, 4- oder 5-Pyrimidyl.

Methylenaminogruppen der Formel IA sind z.B. entsprechend substituierte Guanidino-, über ein Stickstoffatom mit der Butylgruppe verknüpfte Isoharnstoff- oder Isothioharnstoff-, Imidoäther- oder Imidothioäther- und insbesondere Amidinogruppen.

In einem Guanidinorest der Formel IA steht $X_1$ z.B. für Amino, Niederalkylamino, z.B. Methylamino, Diniederalkylamino, z.B. Diäthylamino, Nitroamino, Hydrazino oder Anilino und $X_2$ z.B. für Amino oder Niederalkylamino, z.B. Methylamino. Beispiele für solche Guanidinogruppen sind Guanidino, N-Methyl-, N,N-Dimethyl- oder N,N,N'-Trimethylguanidino, N-Phenylguanidino oder Aminoguanidino.

In einem Isoharnstoff- bzw. Isothioharnstoffrest der Formel Ia steht $X_1$ insbesondere für Amino, Niederalkylamino, z.B. Methylamino, Diniederalkylamino, z.B. Dimethylamino, oder Anilino und $X_2$ z.B. für Niederalkoxy, z.B. Methoxy oder Aethoxy, bzw. für Niederalkylthio, z.B. Methylthio oder Aethylthio. Solche Gruppen sind z.B. die N,N,O-Trimethylisoharnstoff-Gruppe und die N,N-Dimethyl-S-äthyl-, N-Phenyl-S-äthyl- oder N,S-Dimethylisothioharnstoff-Gruppe.

In Imidoäther- bzw. Imidothioäther-Resten der Formel IA ist $X_2$ z.B. Niederalkoxy, z.B. Methoxy oder Aethoxy, oder Benzyloxy, bzw. Niederalkylthio, z.B. Methylthio oder Aethylthio, und $X_1$ hat die gleiche Bedeutung wie $X_2$ oder steht für Wasserstoff, Niederalkyl, z.B. Methyl, oder Phenyl. Entsprechende Gruppen sind z.B. die Methylformamidat-, S-Methylthiobenzimidat-, Methylbenzyloxycarbimidat- oder Diäthyldithiocarbimidat-Gruppe.

In Amidinogruppen der Formel IA steht $X_1$ z.B. für Wasserstoff, Niederalkyl, z.B. Methyl, Aminoniederalkyl, z.B. 2-Aminoäthyl, Niederalkenyl, z.B. Allyl, Phenyl, Thienyl, z.B. 2-Thienyl, Thiazolyl, z.B. 4-Thiazolyl,oder Pyridyl, z.B. 2-, 3- oder 4-Pyridyl, und $X_2$ steht z.B. für Amino, Niederalkylamino, z.B. Methylamino oder Isopropylamino, Diniederalkylamino, z.B. Dimethylamino, oder Niederalkylenamino, z.B. Piperidino. Geeignete Amidinogruppen sind z.B. Benzamidino, 4-Pyridylcarboxamidino, 1-Piperidinylmethylenimino, oder insbesondere Formamidino, Acetamidino, N-Methyl-, N-Isopropyl- oder N,N-Dimethylformamidino.

Die in den Verbindungen der Formel I vorhandenen funktionellen Gruppen, wie Carboxy-, Amino- oder Hydroxygruppen, insbesondere die Aminogruppe $R_1$, die Carboxylgruppen $-C(=O)-R_2$ und die Hydroxygruppe $R_3$ sind gegebenenfalls durch Schutzgruppen geschützt, die in der Penem-, Penicillin-, Cephalosporin- und Peptidchemie verwendet werden.

Solche Schutzgruppen sind leicht, d.h. ohne dass unerwünschte Nebenreaktionen stattfinden, beispielsweise solvolytisch,reduktiv oder auch unter physiologischen Bedingungen, abspaltbar.

Schutzgruppen dieser Art sowie ihre Einführung und Abspaltung sind beispielsweise beschrieben in

BAD ORIGINAL

J.F.W. McOmie, "Protective Groups in Organic Chemistry", Plenum Press, London, New York, 1973,
T.W. Greene, "Protective Groups in Organic Synthesis", Wiley, New York, 1981,
"The Peptides", Vol. I, Schroeder und Luebke, Academic Press, London, New York, 1965 und
Houben-Weyl, "Methoden der Organischen Chemie", Band 15/1, Georg Thieme Verlag, Stuttgart, 1974.

Eine geschützte Aminogruppe $R_1$ kann beispielsweise in Form einer leicht spaltbaren Acylamino-, Acylimino-, verätherten Mercaptoamino-, Silyl- oder Stannylaminogruppe oder als Enamino-, Nitro- oder Azidogruppe vorliegen.

In einer entsprechenden Acylaminogruppe ist Acyl beispielsweise der Acylrest einer organischen Säure mit z.B. bis zu 18 Kohlenstoffatomen, insbesondere einer gegebenenfalls, z.B. durch Halogen oder Phenyl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Fluor-, 2-Brom-, 2-Jod-, 2,2,2-Trifluor- oder 2,2,2-Trichloracetyl, gegebenenfalls substituiertes Benzoyl, z.B. Benzoyl, Halogenbenzoyl, wie 4-Chlorbenzoyl, Niederalkoxybenzoyl, wie 4-Methoxybenzoyl, oder Nitrobenzoyl, wie 4-Nitrobenzoyl. Insbesondere ist auch Niederalkenyloxycarbonyl, z.B. Allyloxycarbonyl, oder gegebenenfalls in 1- oder 2-Stellung substituiertes Niederalkoxycarbonyl geeignet, wie Niederalkoxycarbonyl, z.B. Methoxy- oder Aethoxycarbonyl, gegebenenfalls substituiertes Benzyloxycarbonyl, z.B. Benzyloxycarbonyl oder 4-Nitrobenzyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder 2-(tris-

substituiertes Silyl)-äthoxycarbonyl, wie 2-Triniederalkylsilyläthoxycarbonyl, z.B. 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

In einer Acyliminogruppe ist Acyl beispielsweise der Acylrest einer organischen Dicarbonsäure mit z.B. bis zu 12 Kohlenstoffatomen, insbesondere einer entsprechenden aromatischen Dicarbonsäure, wie Phthalsäure. Eine solche Gruppe ist in erster Linie Phthalimino.

Eine verätherte Mercaptoaminogruppe ist in erster Linie eine gegebenenfalls durch Niederalkyl, wie Methyl oder tert.-Butyl, Niederalkoxy, wie Methoxy, Halogen, wie Chlor oder Brom, und/oder Nitro substituierte Phenylthioaminogruppe oder eine Pyridylthioaminogruppe. Entsprechende Gruppen sind beispielsweise 2- oder 4-Nitrophenylthioamino oder 2-Pyridylthioamino.

Eine Silyl- oder Stannylaminogruppe ist in erster Linie eine organische Silyl- bzw. Stannylaminogruppe, worin das Silicium- bzw. Zinnatom vorzugsweise Niederalkyl, z.B. Methyl, Aethyl, n-Butyl oder tert.-Butyl, ferner Niederalkoxy, z.B. Methoxy, als Substituenten enthält. Entsprechende Silyl- oder Stannylgruppen sind in erster Linie Triniederalkylsilyl, insbesondere Trimethylsilyl, ferner Dimethyl-tert.-butylsilyl, oder entsprechend substituiertes Stannyl, z.B. Tri-n-butylstannyl.Das Siliciumatom einer Silylaminogruppe kann auch nur durch zwei Niederalkylgruppen, z.B. Methylgruppen, und die Amino-, Hydroxy- oder Carboxylgruppe eines zweiten Moleküls der Formel I substituiert sein. Verbindungen mit solchen Schutzgruppen lassen sich z.B. bei Verwendung von Dimethyldichlorsilan als Silylierungsmittel herstellen.

Weitere geschützte Aminogruppen sind z.B. Enaminogruppen, die an der Doppelbindung in 2-Stellung einen elektronenziehenden Substituenten, beispielsweise eine Carbonylgruppe, enthalten. Geschützte Aminogruppen dieser Art sind beispielsweise 1-Acyl-niederalk-1-en-2-yl-amino-Reste, worin Acyl z.B. der entsprechende Rest einer Niederalkancarbonsäure, z.B. Essigsäure, einer gegebenenfalls, z.B. durch Niederalkyl, wie Methyl oder tert. Butyl, Niederalkoxy, wie Methoxy, Halogen, wie Chlor, und/oder Nitro substituierten Benzoesäure, oder insbesondere eines Kohlensäurehalbesters, wie eines Kohlensäureniederalkylhalbesters, z.B. -methylhalbesters oder -äthylhalbesters, und Niederalk-1-en insbesondere 1-Propen bedeutet. Entsprechende geschützte Aminogruppen sind in erster Linie 1-Niederalkanoyl-prop-1-en-2-yl-amino, z.B. 1-Acetyl-prop-1-en-2-yl-amino, oder 1-Niederalkoxycarbonyl-prop-1-en-2-yl-amino, z.B. 1-Aethoxycarbonyl-prop-1-en-2-yl-amino.

In einer geschützten Carboxylgruppe der Formel $-C(=O)-R_2^A$ ist $R_2^A$ insbesondere eine verätherte Hydroxygruppe, welche zusammen mit der Carbonylgruppierung eine vorzugsweise leicht spaltbare, z.B. reduktiv, wie hydrogenolytisch, oder solvolytisch, wie acidolytisch oder insbesondere basisch oder neutral hydrolytisch, oder eine unter physiologischen Bedingungen spaltbare oder leicht in eine andere funktionell abgewandelte Carboxylgruppe, wie in eine andere veresterte Carboxylgruppe umwandelbare, veresterte Carboxylgruppe bildet.

Solche veresterten Carboxylgruppen enthalten als veresternde Gruppen in erster Linie in 1-Stellung verzweigte oder in 1- oder 2-Stellung geeignet substituierte Niederalkylgruppen. Bevorzugte in veresterter Form vorliegende Carboxylgruppen sind unter anderen Niederalkoxycarbonyl, z.B. Methoxycarbonyl, Aethoxycarbonyl, Isopropoxycarbonyl oder tert.-Butoxycarbonyl, und (Hetero-)Arylmethoxycarbonyl mit 1 bis 3 Arylresten oder einem monocyclischen Heteroarylrest, wobei diese gegebenenfalls z.B. durch Niederalkyl, wie tert.-Niederalkyl, z.B. tert.-Butyl, Halogen, z.B. Chlor, und/oder Nitro mono- oder polysubstituiert sind. Beispiele für solche Gruppen sind gegebenenfalls z.B. wie oben erwähnt, substituiertes Benzyloxycarbonyl, z.B. 4-Nitrobenzyloxycarbonyl, gegebenenfalls, z.B. wie oben erwähnt, substituiertes Diphenylmethoxycarbonyl, z.B. Diphenylmethoxycarbonyl, oder Triphenylmethoxycarbonyl, oder gegebenenfalls, z.B. wie oben erwähnt, substituiertes Picolyloxycarbonyl, z.B. 4-Picolyloxycarbonyl, oder Furfuryloxycarbonyl, wie 2-Furfuryloxycarbonyl. Weitere geeignete Gruppen sind Niederalkanoylmethoxycarbonyl, wie Acetonyloxycarbonyl, Aroylmethoxycarbonyl, worin die Aroylgruppe z.B. gegebenenfalls, z.B. durch Halogen, wie Brom, substituiertes Benzoyl darstellt, z.B. Phenacyloxycarbonyl, Halogenniederalkoxycarbonyl, wie 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder ω-Halogenniederalkoxycarbonyl, worin Niederalkoxy 4-7 Kohlenstoffatome enthält, z.B. 4-Chlorbutoxycarbonyl, Phthalimidomethoxycarbonyl, Niederalkenyloxycarbonyl, z.B. Allyloxycarbonyl, oder in 2-Stellung durch Niederalkylsulfonyl, Cyano oder trisubstituiertes Silyl, wie Triniederalkylsilyl oder Triphenylsilyl, substituiertes Aethoxycarbonyl, z.B. 2-Methylsulfonyläthoxycarbonyl, 2-Cyanoäthoxycarbonyl, 2-Trimethylsilyläthoxycarbonyl oder 2-(Di-n-butyl-methyl-silyl)-äthoxycarbonyl.

Weitere in veresterter Form vorliegende geschützte Carboxylgruppen sind entsprechende organische Silyloxycarbonyl-, ferner entsprechende organische Stannyloxycarbonylgruppen. In diesen enthält das Silicium- bzw. Zinnatom vorzugsweise Niederalkyl, insbesondere Methyl oder Aethyl, ferner Niederalkoxy, z.B. Methoxy, als Substituenten. Geeignete Silyl- bzw. Stannylschutzgruppen sind in erster Linie Triniederalkylsilyl, insbesondere Trimethylsilyl oder Dimethyl-tert.-butylsilyl, oder entsprechend substituierte Stannylgruppen, z.B. Tri-n-butylstannyl.

Eine unter physiologischen Bedingungen spaltbare veresterte Carboxylgruppe ist in erster Linie eine Acyloxymethoxycarbonylgruppe, worin Acyl z.B. den Rest einer organischen Carbonsäure, in erster Linie einer gegebenenfalls substituierten Niederalkancarbonsäure bedeutet, oder worin Acyloxymethyl den Rest eines Lactons bildet, 1-Niederalkoxyniederalkoxycarbonyl oder auch 1-Niederalkoxycarbonyloxy-niederalkoxycarbonyl, worin Niederalkyl z.B. Methyl, Propyl, Butyl oder insbesondere Aethyl und Niederalkoxy z.B. Methoxy, Aethoxy, Propoxy oder Butoxy ist. Solche Gruppen sind z.B. Niederalkanoyloxymethoxycarbonyl, z.B. Acetoxymethoxycarbonyl oder Pivaloyloxymethoxycarbonyl, Aminoniederalkanoy -

oxymethoxycarbonyl, insbesondere α-Aminoniederalkanoyloxymethoxycarbonyl, z.B. Glycyloxymethoxycarbonyl, L-Valyloxymethoxycarbonyl, L-Leucyloxymethoxycarbonyl, Phthalidyloxycarbonyl, z.B. 3-Phthalidyloxycarbonyl, 4-Crotonolactonyl oder 4-Butyrolacton-4-yl, Indanyloxycarbonyl, z.B. 5-Indanyloxycarbonyl, 1-Aethoxycarbonyloxyäthoxycarbonyl, Methoxymethoxycarbonyl oder 1-Methoxyäthoxycarbonyl.

In Verbindungen der Formel (I) kann eine Hydroxygruppe $R_3$ beispielsweise durch Acylreste geschützt sein. Geeignete Acylreste sind z.B. gegebenenfalls substituiertes Niederalkanoyl, z.B. Acetyl- oder Trifluoracetyl, gegebenenfalls substituiertes Benzoyl, z.B. Benzoyl, 4-Nitrobenzoyl oder 2,4-Dinitrobenzoyl, gegebenenfalls substituiertes Niederalkoxycarbonyl, z.B. 2-Bromäthoxycarbonyl oder 2,2,2-Trichloräthoxycarbonyl, oder gegebenenfalls substituiertes Phenylniederalkoxycarbonyl, z.B. 4-Nitrobenzyloxycarbonyl. Weitere Hydroxyschutzgruppen sind z.B. trisubstituiertes Silyl, wie Triniederalkylsilyl, z.B. Trimethylsilyl oder tert.-Butyl-dimethylsilyl, 2-Halogenalkylgruppen, z.B. 2-Chlor-, 2-Brom-, 2-Jod- und 2,2,2-Trichloräthyl, und gegebenenfalls substituiertes 1-Phenylniederalkyl, wie gegebenenfalls durch Halogen, z.B. Chlor, Niederalkoxy, z.B. Methoxy, oder Nitro substituiertes Benzyl.

Salze von erfindungsgemässen Verbindungen sind in erster Linie pharmazeutisch verwendbare, nichttoxische Salze, wie diejenigen von Verbindungen der Formel I, worin $R_2$ Hydroxy ist. Solche Salze sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoesäure-2-diäthylaminoäthylester, Niederalkylenaminen, z.B. 1-Aethylpiperidin, Cyclalkylaminen, z.B. Dicyclohexylamin, oder Benzylaminen, z.B. N,N'-Dibenzyläthylendiamin, Dibenzylamin oder

N-Benzyl-β-phenäthylamin. Verbindungen der Formel I mit einer basischen Gruppe, z.B. solche, worin $R_1$ Amino bedeutet, können Säureadditionssalze, z.B. mit anorganischen Säuren, wie Salzsäure, Schwefelsäure oder Phosphorsäure, oder mit geeigneten organischen Carbon- oder Sulfonsäuren, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe, z.B. solche, worin $R_1$ Amino und $R_2$ Hydroxy bedeuten, können auch in Form von inneren Salzen, d.h. in zwitterionischer Form, vorliegen.

In den Penemverbindungen der Formel I können die beiden asymmetrischen Kohlenstoffatome in 5- und 6-Stellung in der R-, der S- oder der racemischen R,S-Konfiguration vorliegen. Bevorzugt sind die Verbindungen, in denen die Konfiguration des 5-Kohlenstoffatoms derjenigen des natürlichen Penicillins entspricht (5R-Konfiguration). Die Wasserstoffatome in 5- und 6-Stellung können in cis- oder bevorzugt in trans-Stellung zueinander stehen. In der bevorzugten Konfiguration nimmt der 6-Hydroxymethyl-Substituent die S-Konfiguration ein.

Die Verbindungen der vorliegenden Erfindung weisen wertvolle pharmakologische Eigenschaften auf, oder können als Zwischenprodukte zur Herstellung von solchen Verbindungen mit wertvollen pharmakologischen Eigenschaften verwendet werden. Verbindungen der Formel I, worin $R_1$ eine Aminogruppe oder eine gegebenenfalls substituierte Methylenaminogruppe A darstellt, $R_2$ Hydroxy oder zusammen mit der Carbonylgruppe eine unter physiologischen Bedingungen spaltbare veresterte Carboxylgruppe bedeutet, und $R_3$ Hydroxy ist, oder pharmakologisch verwendbare Salze von solchen Verbindungen mit salzbildenden Gruppen haben antibakterielle Wirkungen. Beispielsweise sind sie in vitro gegen

grampositive und gramnegative Erreger, z.B. Staphylococcus aureus, Streptococcus pyogenes, Streptococcus pneumoniae, Streptococcus faecalis, Neisseria menigitidis, Neisseria gonorrhoeae, Enterobacteriaceae, Haemophilus influenzae, Pseudomonas aeruginosa und Bacteroides sp. in minimalen Konzentrationen von ca. 0,5 bis ca. 64 µg/ml wirksam. In vivo, bei der systemischen Infektion der Maus, z.B. durch Staphylococcus aureus oder Streptococcus pyogenes, ergibt sich bei oraler Applikation erfindungsgemässer Verbindungen ein minimal wirksamer Dosisbereich von ca. 0,65 bis ca. 3 mg/kg.

In dem folgenden Versuchsbericht wird anhand von ausgewählten Verbindungen die Wirksamkeit von Verbindungen der Formel I gezeigt:

Versuchsbericht:

I. Getestete Verbindungen:

Die antibiotische Wirksamkeit der folgenden Verbindungen wurde getestet:

1. trans-2-(4-Aminobutyl)-6-hydroxymethyl-penem-3-carbonsäure (Beispiel 19)

2. (5R, 6S)-2-(4-Aminobutyl)-6-hydroxymethyl-penem-3-carbonsäure (Beispiel 28)

II. Experimentelles:

A. Die antibiotische Aktivität der Testverbindungen in vitro wurde durch die Agarverdünnungsmethode nach Ericsson, H.M., und Sherris, S.C., 1971, Acta Path. Microb. Scand. Section B, Suppl. No. 217, Bd. 1-90, in DST Agar ermittelt. Die gefundenen, das Wachstum der Testorganismen noch hemmenden Minimalkonzentrationen (MIC = minimum inhibitory concentrations) werden in Mikrogramm pro Milliliter (µg/ml) für die geprüften Verbindungen in der Tabelle I angegeben.

B. Die chemotherapeutische Wirksamkeit in vivo gegen systemische Infektionen in weiblichen SPF, $MF_2$ Mäusen wurde nach der Methode von Zak, O., et al., 1979, Drugs Exptl. Clin, Res. 5, 45-59, ermittelt. Die gefundenen $ED_{50}$-Werte in Milligramm Substanz pro Kilogramm Maus (mg/kg) für eine Anzahl von Mikroorganismen werden für die subcutan (s.c) bzw. oral (p.o.) applizierten Testverbindungen in der Tabelle 2 angegeben.

III. Versuchsergebnisse:

Tabelle 1: Antibiotische Wirksamkeit (in vitro)

| Mikroorganismus | Testverbindung MIC (μg/ml) | |
|---|---|---|
| | 1 | 2 |
| Staphylococcus aureus 10 B | 0,2 | 0,5 |
| St. aureus 2999 $i^+$ $p^+$ | 0,2 | 0,5 |
| St. aureus 568 | 0,1 | 0,5 |
| Streptococcus pyogenes Aronson | 0,2 | 0,5 |
| St. pneumoniae III/84 | 0,1 | 0,5 |
| St. faecalis 1362/3 | 64 | 16 |
| Neisseria meningitidis 1316 | 1 | |
| N. gonorrhoeae 1317/4 | 0,2 | |
| Haemophilus influenzae NCTC 4560 | 32 | |
| Escherichia coli 205 | 32 | 4 |
| E. coli 16 $R^+$ TEM | 32 | 4 |
| E. coli 576 | 32 | 4 |
| Klebsiella pneumoniae 327 | 32 | 4 |
| Serratia marcescens 344 | 32 | 16 |
| Enterobacter cloacae P 99 | 32 | 4 |
| Proteus mirabilis 774 | 32 | 16 |
| P. rettgeri 856 | 128 | 64 |

Tabelle 1: (Fortsetzung)

| Mikroorganismus | Testverbindung MIC (µg/ml) 1 | 2 |
|---|---|---|
| Morganella morganii 2359 | 32 | 16 |
| Pseudomonas aeruginosa ATCC 12055 | 32 | 8 |
| Bacteroides fragilis Lo 1 | 1 | 0.5 |
| B. fragilis 17390 | 1 | |
| B. thetaiotaomicron E 50 | 2 | |
| B. distanonis Am 9 | 8 | |
| B. vulgatus Am5 | 0,5 | |
| Spherophorus varius Am 7 | 0,25 | |
| Fusobacterium fusiforme T3/18 | 0,12 | |

Tabelle 2: Chemotherapeutische Wirksamkeit (in vivo)

| Mikroorganismus | $ED_{50}$ (mg/kg) 1 | | 2 | |
|---|---|---|---|---|
| | s.c. | p.o. | s.c. | p.o. |
| Staphylococcus aureus 10 B | 6 | 5,2 | 4 | 3 |
| St. aureus 2999 $i^+$ $p^+$ | 10 | | | |
| Streptococcus pyogenes Aronson | 1,7 | 1,5 | 1,2 | 0,9 |

Die neuen Verbindungen können deshalb als oral oder parenteral applizierbare antibakterielle Antibiotika, z.B. in Form von entsprechenden pharmazeutischen Präparaten, zur Behandlung von Infektionen Verwendung finden.

Verbindungen der Formel I, worin mindestens eine der vorhandenen funktionellen Gruppen in geschützter Form vorliegt, wobei eine geschützte Carboxylgruppe von einer physiologisch spaltbaren veresterten Carboxylgruppe verschieden ist, können als Zwischenprodukte zur Herstellung der oben genannten pharmakologisch wirksamen Verbindungen der Formel I verwendet werden.

Die Erfindung betrifft insbesondere Verbindungen der Formel I, worin $R_1$ Amino, 1-Acyl-niederalk-1-en-2-yl-amino oder eine mono- oder disubstituierte Methylenaminogruppe bedeutet, $R_2$ Hydroxy oder zusammen mit der Carbonylgruppe eine unter physiologischen Bedingungen spaltbare veresterte Carboxylgruppe darstellt und $R_3$ Hydroxy ist, ihre Salze, ihre optischen Isomeren und Mischungen ihrer optischen Isomeren sowie ihre geschützten Derivate.

Die Erfindung betrifft vor allem Verbindungen der Formel I, worin $R_1$ Amino ist, $R_2$ Hydroxy, Niederalkanoyloxymethoxy, Aminoniederalkanoyloxymethoxy, Phthalidyloxy, 4-Crotonolactonyloxy, 4-Butyrolacton-4-yloxy, Indanyloxy, 1-Niederalkoxy-niederalkoxy oder 1-Niederalkoxycarbonyloxy-niederalkoxy darstellt und $R_3$ Hydroxy ist, ihre Salze, insbesondere ihre pharmazeutisch verwendbaren Salze, ihre optischen Isomeren und Mischungen ihrer optischen Isomeren.

Hervorzuheben ist die Verbindung der Formel I, worin $R_1$ Amino darstellt, $R_2$ Hydroxy bedeutet und $R_3$ Hydroxy ist, ihre optischen Isomeren, insbesondere das 5R,6S-Isomere, und ihre pharmazeutisch verwendbaren Salze.

Die Erfindung betrifft insbesondere die in den Beispielen genannten Verbindungen der Formel I und deren pharmazeutisch verwendbare Salze.

Die Verbindungen der vorliegenden Erfindung werden nach an sich bekannten Verfahren hergestellt.

Die neuen Verbindungen werden hergestellt, indem man eine Ylid-Verbindung der Formel

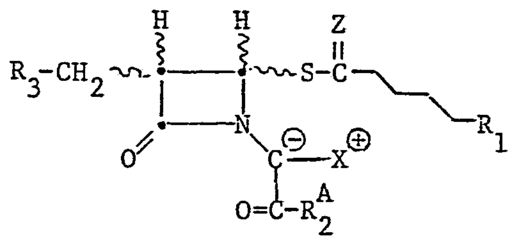

(II)

worin $R_1$ eine geschützte Aminogruppe ist, Z Sauerstoff oder Schwefel bedeutet, $X^{\oplus}$ entweder eine dreifach substituierte Phosphoniogruppe oder eine zweifach veresterte Phosphonogruppe zusammen mit einem Kation bedeutet, $R_2^A$ die oben angegebene Bedeutung hat und $R_3$ eine gegebenenfalls geschützte Hydroxygruppe ist, ringschliesst, und, wenn erwünscht oder notwendig, in einer erhältlichen Verbindung der Formel I eine geschützte Aminogruppe $R_1$ in die freie oder in eine andere geschützte Aminogruppe $R_1$ überführt, und/oder, wenn erwünscht, in einer erhältlichen Verbindung der Formel I eine geschützte Carboxylgruppe $-C(=O)-R_2$ in die freie oder in eine andere geschützte Carboxylgruppe $-C(=O)-R_2$ überführt, und/oder, wenn erwünscht, in einer erhältlichen Verbindung der Formel I, worin $R_1$ Amino bedeutet, $R_1$ in eine substituierte Aminogruppe überführt, und/oder, wenn erforderlich, eine geschützte Hydroxygruppe $R_3$ in die freie Hydroxygruppe $R_3$ oder eine freie Hydroxygruppe $R_3$ in eine geschützte Hydroxygruppe $R_3$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomeren auftrennt.

Die Gruppe $X^{\oplus}$ im Ausgangsmaterial der Formel II ist eine der bei Wittig-Kondensationsreaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetall-, z.B. Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. Natriumion.

Die Ylid-Verbindungen der Formel II werden in der isomeren Ylen-Form auch als Phosphoran-Verbindungen bezeichnet. In Phosphonio-Verbindungen der Formel II wird die negative Ladung durch die positiv geladene Phosphoniogruppe neutralisiert. In Phosphono-Verbindungen der Formel II wird die negative Ladung durch das Kation einer starken Base neutralisiert, das je nach Herstellungsweise des Phosphono-Ausgangsmaterials z.B. ein Alkalimetall-, z.B. Natrium-, Lithium- oder Kaliumion, sein kann. Die Phosphono-Ausgangsstoffe werden daher als Salze in die Reaktion eingesetzt.

Der Ringschluss kann spontan, d.h. bei der Herstellung der Ausgangsstoffe, oder durch Erwärmen, z.B. in einem Temperaturbereich von etwa 30°C bis 160°C, vorzugsweise von etwa 50°C bis etwa 100°C, erfolgen. Die Reaktion wird vorzugsweise in einem geeigneten inerten Lösungsmittel, wie in einem aliphatischen, cycloaliphatischen oder aromatischen Kohlenwasserstoff, z.B. Hexan, Cyclohexan, Benzol oder Toluol, einem halogenierten Kohlenwasserstoff, z.B. Methylenchlorid, einem Aether, z.B. Diäthyläther, Dimethoxyäthan oder Diäthylenglykoldimethyläther, einem cyclischen Aether, z.B. Dioxan oder Tetrahydrofuran, einem Carbonsäureamid, z.B. Dimethylformamid, einem Diniederalkylsulfoxid, z.B. Dimethylsulfoxid, oder einem Niederalkanol, z.B. Methanol, Aethanol oder tert.-Butanol, oder in einem Gemisch davon, und, falls notwendig, in einer Inertgas-, z.B. Stickstoffatmosphäre, durchgeführt.

Eine Ausgangsverbindung der Formel II, worin $X^{\oplus}$ eine Phosphonogruppe zusammen mit einem Kation ist, wird vorzugsweise in situ hergestellt, indem man eine Verbindung der Formel

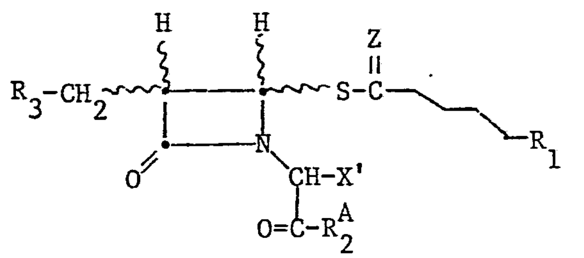

(IIa) ,

worin X' eine Phosphonogruppe bedeutet, mit einem geeigneten basischen Reagenz, wie einer anorganischen Base, z.B. einem Alkalimetallcarbonat, wie Natrium- oder Kaliumcarbonat, oder einer organischen Base, wie einem Triniederalkylamin, z.B. Triäthylamin, oder einer cyclischen Base vom Amidintyp, wie einer entsprechenden Diazabicycloalkenverbindung, z.B. 1,5-Diazabicyclo[5.4.0]undec-5-en, behandelt.

Bevorzugt werden solche Ausgangsmaterialien der Formel II verwendet, die zu den eingangs als besonders bevorzugt genannten Verbindungen der Formel I führen, beispielsweise Verbindungen der Formel II, die eine 3S,4R-Konfiguration aufweisen.

Zur Herstellung von Verbindungen der Formel I, die eine freie Aminogruppe $R_1$ und/oder eine freie Carboxylgruppe $-C(=O)-R_2$ und/oder freie Hydroxygruppe $R_3$ enthalten, werden bevorzugt solche Hydroxy-, Amino- und/oder Carboxylschutzgruppen verwendet, die sich in einem Reaktionsschritt, beispielsweise reduktiv, wie hydrogenolytisch, abspalten lassen.

In einer erfindungsgemäss erhältlichen Verbindung der Formel I mit einer geschützten Aminogruppe $R_1$ kann diese in an sich bekannter Weise, z.B. je nach Art der Schutzgruppe, vorzugsweise mittels Solvolyse oder Reduktion, in die freie Aminogruppe $R_1$ übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino durch Behandeln mit einem geeigneten chemischen Reduktionsmittel, wie Zink in Gegenwart einer geeigneten Carbonsäure, wie wässriger Essigsäure, oder katalytisch mit Wasserstoff in Gegenwart eines Palladiumkatalysators gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z.B. Natriumdithionit gespalten werden. Gegebenenfalls substituiertes Benzyloxycarbonylamino kann z.B. mittels Hydrogenolyse, d.h. durch Behandeln mit Wasserstoff in Gegenwart eines geeigneten Hydrierkatalysators, wie eines Palladiumkatalysators, und Allyloxycarbonylamin, durch Umsetzen mit einer Verbindung des Palladiums, z.B. Tetrakis(triphenylphosphin)palladium, in Gegenwart von Triphenylphosphin und Behandeln mit einer Carbonsäure, z.B. 2-Aethylhexansäure, oder einem Salz davon, gespalten werden. Eine mit einer organischen Silyl- oder Stannylgruppe geschützte Aminogruppe kann z.B. mittels Hydrolyse oder Alkoholyse, eine durch 2-Halogenniederalkanoyl, z.B. 2-Chloracetyl, geschützte Aminogruppe durch Behandeln mit Thioharnstoff in Gegenwart einer Base oder mit einem Thiolatsalz, wie einem Alkalimetallthiolat, des Thioharnstoffs und anschliessender Solvolyse, wie Alkoholyse oder Hydrolyse des entstandenen Kondensationsproduktes freigesetzt werden. Eine durch 2-substituiertes Silyläthoxycarbonyl geschützte Aminogruppe kann durch Behandeln mit einem Fluoridanionen liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetraäthylammoniumfluorid, in die freie Aminogruppe übergeführt werden. Eine in Form einer Azido- oder Nitrogruppe geschützte Aminogruppe wird z.B. durch Reduktion in freies Amino überführt, beispielsweise durch katalytische Hydrierung mit Wasserstoff in Gegenwart eines Hydrierkatalysators wie Platinoxid, Palladium oder Raney-Nickel, oder durch Behandeln mit Zink in Gegenwart einer Säure, wie Essigsäure. Eine in Form einer Phthalimidogruppe geschützte Aminogruppe kann durch Umsetzen mit Hydrazin in die freie Aminogruppe überführt werden. Weiterhin kann eine Arylthioaminogruppe durch Behandeln mit einem nucleophilen Reagenz, wie schwefliger Säure, in Amino umgewandelt werden.

Ferner kann man eine freie Aminogruppe $R_1$ in an sich bekannter Weise in eine substituierte Aminogruppe überführen. So kann man beispielsweise Amino durch Umsetzen mit einem entsprechenden Acylhalogenid, wie -chlorid, in Acylamino $R_1$ und mit einer β-Dicarbonylverbindung, wie einem 1-Niederalkanoyl-aceton oder einem Acetessigsäure-niederalkylester in 1-Niederalkanoyl- bzw. 1-Niederalkoxycarbonylprop-1-en-2-ylamino überführen. Die Umwandlung von Aminogruppen in Amidino-, Guanidino-, Isoharnstoff-, Isothioharnstoff-, Imidoäther- und Imidothioäthergruppen kann beispielsweise nach einem der in der Deutschen Offenlegungsschrift Nr. 26 52 679 genannten Verfahren durchgeführt werden. So können beispielsweise Verbindungen der Formel I, worin $R_1$ Amino darstellt, durch Umsetzen mit Trimethylsilylchlorid und einem Imidohalogenid der Formel $[(X_1,Y_1)C=X_2]^{\oplus}Y_2^{\ominus}$, worin $Y_1$ Halogen, z.B. Chlor, und $Y_2$ ein Anion, z.B. Chlorid, bedeutet, in Amidine und durch Umsetzen mit einem substituierten Isoharnstoff bzw. Isothioharnstoff der Formel $(X_1Y_3)C=X_2$, worin $Y_3$ Niederalkoxy oder Niederalkylthio ist, in Guanidine übergeführt werden.

Eine verfahrensgemäss erhältliche Verbindung der Formel I, worin $R_2$ einen zusammen mit der Carbonylgruppe -C(=O)- eine geschützte Carboxylgruppe bildenden Rest $R_2^A$ bedeutet, kann in an sich bekannter Weise in eine Verbindung der Formel I überführt werden, worin $R_2$ Hydroxy ist. So kann man tert.-Niederalkoxycarbonyl oder in 2-Stellung durch eine trisubstituierte Silylgruppe oder in 1-Stellung durch Niederalkoxy substituiertes Niederalkoxycarbonyl oder gegebenenfalls substituiertes Diphenylmethoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, oder Metallsalz, wie einem Chrom-II-Salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, Ameisensäure oder Glykolsäure, oder eines Alkohols oder Thiols, wobei man vorzugsweise Wasser zugibt, in freies Carboxyl überführen. Die Abspaltung einer Allylschutzgruppe kann z.B. durch Umsetzen mit einer Verbindung des Palladiums, z.B. Tetrakis(triphenylphosphin)palladium, in Gegenwart von Triphenylphosphin und unter Zusatz einer Carbonsäure, z.B. 2-Aethylhexansäure, oder einem Salz davon erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende

2-Jodniederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagenz, wie Natriumthiophenolat oder Natriumjodid gespalten werden kann. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetraäthylammoniumfluorid, in freies Carboxyl überführt werden. Mit einer organischen Silyl- oder Stannylgruppe, wie Triniederalkylsilyl oder -stannyl, verestertes Carboxyl kann in üblicher Weise solvolytisch, z.B. durch Behandeln mit Wasser oder einem Alkohol freigesetzt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall- oder Erdalkalimetallhydroxid oder -carbonat, z.B. Natrium- oder Kaliumhydroxid oder Natrium- oder Kaliumcarbonat, in freies Carboxyl übergeführt werden.

Andererseits können auch Verbindungen der Formel I, worin $R_2$ Hydroxy bedeutet, in Verbindungen der Formel I überführt werden, worin $R_2$ einen zusammen mit der Carbonylgruppe -C(=O)- eine geschützte Carboxylgruppe bildenden Rest $R_2^A$, insbesondere eine veresterte Carboxylgruppe, darstellt. So kann man die freie Carboxylgruppe z.B. durch Behandeln mit einer geeigneten Diazoverbindung, wie einem Diazoniederalkan, z.B. Diazomethan, oder einem Phenyldiazoniederalkan, z.B. Diphenyldiazomethan, wenn notwendig, in Gegenwart einer Lewis-Säure, wie z.B. Bortrifluorid, oder durch Umsetzen mit einem zur Veresterung geeigneten Alkohol in Gegenwart eines Veresterungsmittels, wie eines Carbodiimids, z.B. Dicyclohexylcarbodiimid, sowie Carbonyldiimidazol, verestern. Ester können auch durch Umsetzung eines gegebenenfalls in situ hergestellten Salzes der Säure

mit einem reaktionsfähigen Ester eines Alkohols und einer starken anorganischen Säure, wie Schwefelsäure, oder einer starken organischen Sulfonsäure, wie 4-Toluolsulfonsäure, hergestellt werden. Ferner können Säurehalogenide, wie Chloride (hergestellt z.B. durch Behandeln mit Oxalylchlorid), aktivierte Ester, (gebildet z.B. mit N-Hydroxystickstoffverbindungen, wie N-Hydroxysuccinimid) oder gemischte Anhydride (erhalten z.B. mit Halogenameisensäure-niederalkylestern, wie Chlorameisensäureäthyl- oder Chlorameisensäureisobutylester, oder mit Halogenessigsäurehalogeniden, wie Trichloracetylchlorid) durch Umsetzen mit Alkoholen, gegebenenfalls in Gegenwart einer Base, wie Pyridin, in eine veresterte Carboxylgruppe übergeführt werden.

In einer Verbindung der Formel I mit einer veresterten Carboxylgruppe kann diese in eine andere veresterte Carboxylgruppe überführt werden, z.B. 2-Chloräthoxycarbonyl oder 2-Bromäthoxycarbonyl durch Behandeln mit einem Jodsalz, z.B. Natriumjodid, in 2-Jodäthoxycarbonyl. Ferner kann man in Verbindungen der Formel I, die eine in veresterter Form geschützte Carbonylgruppe enthalten, die Carboxylgruppe wie oben beschrieben abspalten und eine entstandene Verbindung der Formel I mit einer freien Carboxylgruppe oder ein Salz davon durch Umsetzen mit dem reaktionsfähigen Ester eines entsprechenden Alkohols in eine Verbindung der Formel I überführen, worin $R_2^A$ zusammen mit der Carbonylgruppe eine unter physiologischen Bedingungen spaltbare veresterte Carboxylgruppe darstellt.

In verfahrensgemäss erhältlichen Verbindungen der Formel I, worin $R_3$ eine geschützte Hydroxygruppe darstellt, kann diese in an sich bekannter Weise in die freie Hydroxygruppe $R_3$ überführt werden. Beispielsweise wird eine durch eine geeignete Acylgruppe oder eine organische Silyl- oder Stannylgruppe geschützte Hydroxygruppe wie eine entsprechend geschützte Aminogruppe freigesetzt. Eine 2-Halogenniederalkylgruppe und eine gegebenenfalls substituierte Benzylgruppe werden reduktiv abgespalten.

BAD ORIGINAL

Weiterhin kann eine verfahrensgemäss erhältliche Verbindung der Formel I, worin $R_3$ Hydroxy ist, in an sich bekannter Weise in eine Verbindung der Formel I überführt werden, worin $R_3$ eine geschützte Hydroxygruppe ist. So kann man Hydroxy durch Umsetzen mit einem Acylhalogenid, z.B. einem Halogenid, wie dem Chlorid, einer gegebenenfalls substituierten Niederalkancarbonsäure, gegebenenfalls substituierten Benzoesäure oder eines gegebenenfalls substituierten Niederalkyl- oder Phenylniederalkylhalbesters der Kohlensäure, in gegebenenfalls substituiertes Niederalkanoyloxy, Benzoyloxy, Niederalkoxycarbonyloxy oder Phenylniederalkoxycarbonyloxy überführen. Ferner kann man durch Umsetzen mit trisubstituiertem Silylhalogenid bzw. gegebenenfalls substituiertem 1-Phenylniederalkylhalogenid Verbindungen der Formel I erhalten, worin $R_3$ trisubstituiertes Silyloxy bzw. gegebenenfalls substituiertes 1-Phenylniederalkoxy bedeutet.

Salze von Verbindungen der Formel I mit salzbildenden Gruppen können in an sich bekannter Weise hergestellt werden. So kann man Salze von Verbindungen der Formel I mit einer freien Carboxylgruppe, z.B. durch Behandeln mit Metallverbindungen, wie Alkalimetallsalzen von geeigneten organischen Carbonsäuren, z.B. dem Natriumsalz der α-Aethylcapronsäure, oder mit anorganischen Alkali- oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I, worin z.B. $R_1$ Amino und $R_2$ Hydroxy bedeutet, können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Erhaltene Gemische von Isomeren können nach an sich bekannten Methoden in die einzelnen Isomeren aufgetrennt werden, Gemische von diastereomeren Isomeren z.B. durch fraktioniertes Kristallisieren, Adsorptionschromatographie (Kolonnen- oder Dünnschichtchromatographie) oder andere geeignete Trennverfahren.

Die Spaltung von erhaltenen Racematen in ihre optischen Antipoden kann auf verschiedenen Wegen erfolgen.

Einer dieser Wege besteht darin, dass man ein Racemat mit einem optisch aktiven Hilfsstoff reagieren lässt, das dabei entstandene Gemisch zweier diastereomerer Verbindungen mit Hilfe von geeigneten physikalisch-chemischen Methoden trennt und die einzelnen diastereomeren Verbindungen dann in die optisch aktiven Verbindungen spaltet.

Zur Trennung in Antipoden besonders geeignete Racemate sind solche, die eine saure Gruppe besitzen, wie z.B. Racemate von Verbindungen der Formel I, worin $R_2$ Hydroxy ist. Diese sauren Racemate können mit optisch aktiven Basen, z.B. Estern von optisch aktiven Aminosäuren, oder (-)-Brucin, (+)-Chinidin, (-)-Chinin, (+)-Cinchonin, (+)-Dehydroabietylamin, (+)- und (-)-Ephedrin, (+)- und (-)-1-Phenyläthylamin oder deren N-mono- oder N,N-dialkylierten Derivaten zu Gemischen, bestehend aus zwei diastereomeren Salzen, umgesetzt werden.

In Carboxylgruppen enthaltenden Racematen kann diese Carboxylgruppe auch durch einen optisch aktiven Alkohol, wie (-)-Menthol, (+)-Borneol,

(+)- oder (-)-2-Octanol verestert sein oder werden, worauf nach erfolgter Isolierung des gewünschten Diastereomeren die Carboxylgruppe freigesetzt wird.

Zur Racemattrennung kann auch die Hydroxygruppe mit optisch aktiven Säuren oder deren reaktionsfähigen, funktionellen Derivaten verestert werden, wobei sich diastereomere Ester bilden. Solche Säuren sind beispielsweise (-)-Abietinsäure, D(+)- und L(-)-Aepfelsäure, N-acylierte optisch aktive Aminosäure, (+)- und (-)-Camphansäure, (+)- und (-)-Ketopinsäure, L(+)-Ascorbinsäure, (+)-Camphersäure, (+)-Campher-10-sulfonsäure(β), (+)- oder (-)-α-Bromcampher-π-sulfonsäure, D(-)-Chinasäure, D(-)-Isoascorbinsäure, D(-)- und L(+)-Mandelsäure, (+)-1-Menthoxyessigsäure, D(-)- und L(+)-Weinsäure und deren Di-O-benzoyl- und Di-O-p-toluylderivate.

Durch Umsetzung mit optisch aktiven Isocyanaten, wie mit (+)- oder (-)-1-Phenyläthylisocyanat, können Verbindungen der Formel (I), worin $R_1$ geschütztes Amino und $R_2$ einen Rest $R_2^A$ bedeutet, in ein Gemisch diastereomerer Urethane umgewandelt werden.

Basische Racemate, z.B. Verbindungen der Formel I, worin $R_1$ Amino, ist, können mit den genannten optischen aktiven Säuren diastereomere Salze bilden.

Die Spaltung der aufgetrennten Diastereomeren in die optisch aktiven Verbindungen der Formel I erfolgt ebenfalls nach üblichen Methoden. Aus den Salzen befreit man die Säuren oder die Basen z.B. durch Behandeln mit stärkeren Säuren bzw. Basen als die ursprünglich eingesetzten. Aus den Estern und Urethanen erhält man die gewünschten optisch aktiven Verbindungen beispielsweise nach alkalischer Hydrolyse oder nach Reduktion mit einem komplexen Hydrid, wie Lithiumaluminiumhydrid.

Eine weitere Methode zur Auftrennung der Racemate besteht in der Chromatographie an optisch aktiven Absorptionsschichten, beispielsweise an Rohrzucker.

Nach einer dritten Methode können die Racemate in optisch aktiven Lösungsmitteln gelöst und der schwerer lösliche optische Antipode auskristallisiert werden.

Bei einer vierten Methode benützt man die verschiedene Reaktionsfähigkeit der optischen Antipoden gegenüber biologischem Material wie Mikroorganismen oder isolierten Enzymen.

Nach einer fünften Methode löst man die Racemate und kristallisiert einen der optischen Antipoden durch Animpfen mit einer kleinen Menge eines nach den obigen Methoden erhaltenen optisch aktiven Produktes aus.

Die Auftrennung von Diastereomeren in die einzelnen Racemate und der Racemate in die optischen Antipoden, kann auf einer beliebigen Verfahrensstufe, d.h. z.B. auch auf der Stufe des Ausgangsmaterials der Formel II oder auf einer beliebigen Stufe des nachstehend beschriebenen Verfahrens zur Herstellung des Ausgangsmaterials der Formel II, durchgeführt werden.

Bei allen nachträglichen Umwandlungen erhaltener Verbindungen der Formel I werden solche Reaktionen bevorzugt, die unter neutralen, alkalischen oder schwach sauren Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen

wird. Ferner können Ausgangsstoffe in Form von Derivaten verwendet oder in situ, gegebenenfalls unter den Reaktionsbedingungen, gebildet werden. Beispielsweise kann ein Ausgangsmaterial der Formel II, worin Z Sauerstoff ist, aus einer Verbindung der Formel II, worin Z eine, wie nachstehend beschrieben, gegebenenfalls substituierte Methylidengruppe ist, durch Ozonisierung und anschliessende Reduktion des gebildeten Ozonids, analog dem weiter unten angegebenen Verfahren (Stufe 3.3), in situ hergestellt werden, worauf in der Reaktionslösung die Cyclisierung zur Verbindung der Formel I erfolgt.

Die Ausgangsverbindungen der Formel II und die Vorstufen können, wie in dem Reaktionsschemata I, II und III angegeben, hergestellt werden:

Reaktionsschema I

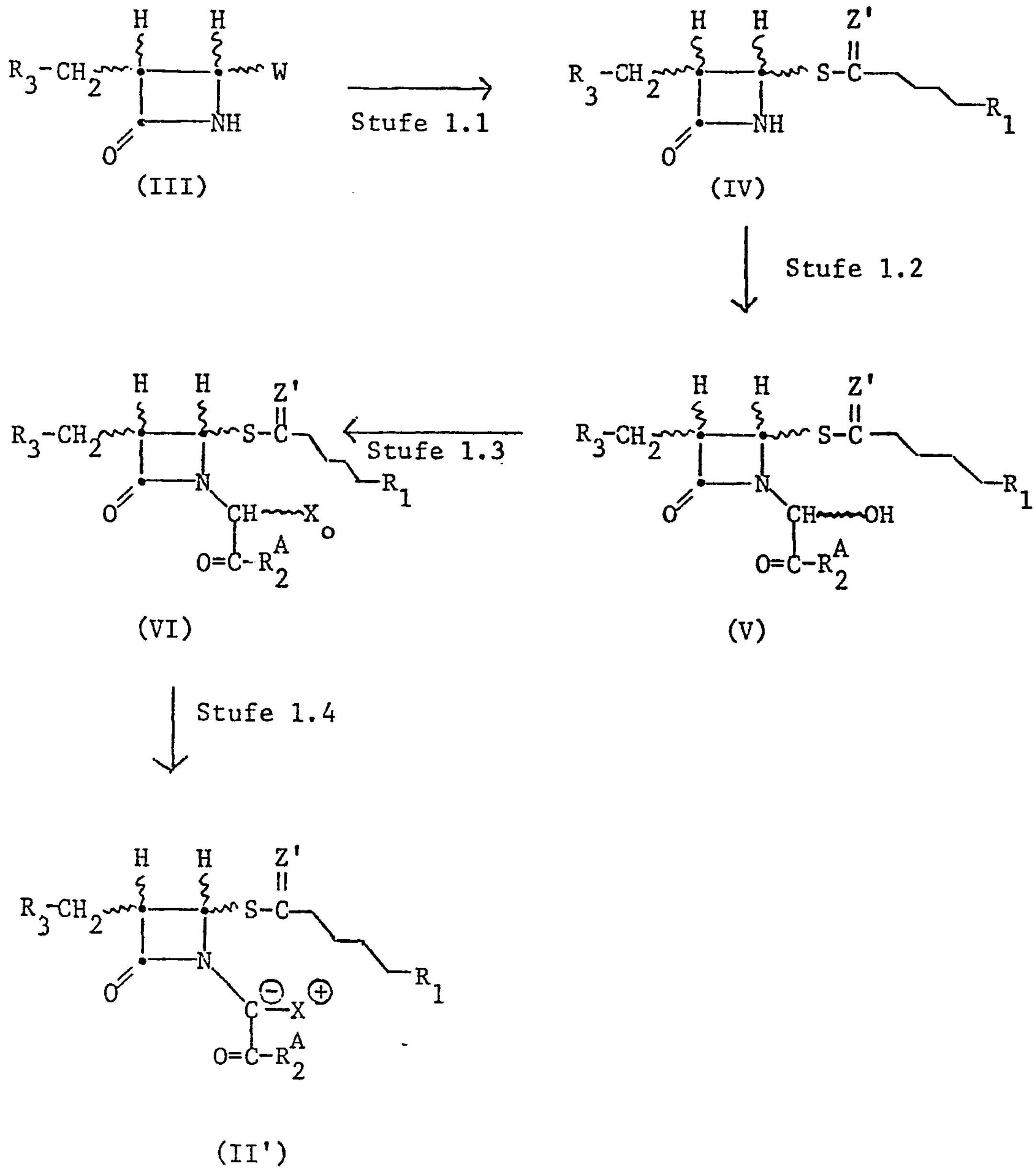

In den Verbindungen der Formeln IV, V, VI und II' ist Z' Sauerstoff, Schwefel oder auch eine gegebenenfalls durch einen oder zwei Substituenten Y substituierte Methylidengruppe, die durch Oxidation in eine Oxogruppe Z überführt werden kann. Ein Substituent Y dieser Methylidengruppe ist ein organischer Rest, beispielsweise gegebenenfalls substituiertes Niederalkyl, z.B. Methyl oder Aethyl, Cycloalkyl, z.B. Cyclopentyl oder Cyclohexyl, Phenyl oder Phenylniederalkyl, z.B. Benzyl, oder insbesondere eine, inklusive mit einem optisch aktiven Alkohol, wie l-Menthol, veresterte Carboxylgruppe, z.B. einer der unter $R_2^A$ erwähnten gegebenenfalls substituierten Niederalkoxycarbonyl- oder Arylmethoxycarbonylreste oder auch l-Menthyloxycarbonyl. Diese Methylidengruppe trägt bevorzugt einen der genannten Substituenten. Hervorzuheben sind die Methoxycarbonylmethyliden-, Aethoxycarbonylmethyliden- und die l-Menthyloxycarbonylmethylidengruppe Z'. Letztere kann zur Herstellung optisch aktiver Verbindungen der Formeln IV bis VI und II verwendet werden.

In den Verbindungen der Formeln III-VI sowie II' ist der Rest $R_3$ bevorzugt eine der genannten geschützten Hydroxygruppen, z.B. gegebenenfalls substituiertes 1-Phenylniederalkoxy, gegebenenfalls substituiertes Phenylniederalkoxycarbonyloxy, oder trisubstituiertes Silyloxy, und $R_1$ bevorzugt substituiertes Amino.

Stufe 1.1: Ein Thio-azetidinon der Formel IV wird erhalten, indem man ein 4-W-Azetidinon der Formel III, worin W eine nucleofuge Abgangsgruppe bedeutet, mit einer Mercaptoverbindung der Formel

$$R_1-(CH_2)_4-C(=Z')-SH$$

oder einem Salz, z.B. einem Alkalimetall-, wie Natrium- oder Kaliumsalz davon, behandelt, und, wenn gewünscht, in einer erhältlichen Verbindung der Formel IV, worin $R_3$ Hydroxy ist, Hydroxy in geschütztes Hydroxy überführt.

Die nucleofuge Abgangsgruppe W in einem Ausgangsmaterial der Formel III ist ein durch den nucleophilen Rest

$R_1-(CH_2)_4-C(=Z')-S-$ ersetzbarer Rest. Solche Gruppen W sind beispielsweise Acyloxyreste, Sulfonylreste $R_o-SO_2-$, worin $R_o$ ein organischer Rest ist, Azido oder Halogen. In einem Acyloxyrest W ist Acyl z.B. der Rest einer organischen Carbonsäure, inklusive einer optisch aktiven Carbonsäure, und bedeutet beispielsweise Niederalkanoyl, z.B. Acetyl oder Propionyl, gegebenenfalls substituiertes Benzoyl, z.B. Benzoyl oder 2,4-Dinitrobenzoyl, Phenylniederalkanoyl, z.B. Phenylacetyl, oder den Acylrest einer der oben genannten optisch aktiven Säuren. In einem Sulfonylrest $R_o-SO_2-$ ist $R_o$ beispielsweise gegebenenfalls durch Hydroxy substituiertes Niederalkyl, wie Methyl, Aethyl oder 2-Hydroxyäthyl, ferner auch entsprechendes substituiertes optisch aktives Niederalkyl, z.B. (2R)- oder (2S)-1-Hydroxyprop-2-yl, durch einen optisch aktiven Rest substituiertes Methyl, wie Campheryl, oder Benzyl, oder gegebenenfalls substituiertes Phenyl, wie Phenyl, 4-Bromphenyl oder 4-Methylphenyl. Ein Halogenrest W ist z.B. Brom, Jod oder insbesondere Chlor. W ist bevorzugt Methyl- oder 2-Hydroxyäthylsulfonyl, Acetoxy oder Chlor.

Die nucleophile Substitution kann unter neutralen oder schwach basischen Bedingungen in Gegenwart von Wasser und gegebenenfalls einem mit Wasser mischbaren organischen Lösungsmittel durchgeführt werden. Die basischen Bedingungen können beispielsweise durch Zugabe einer anorganischen Base, wie eines Alkalimetall- oder Erdalkalimetallhydroxids, -carbonats oder -hydogencarbonats, z.B. von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, wie Dimethylformamid, Acetonitril und ähnliche verwendet werden. Die Reaktion wird üblicherweise bei Raumtemperatur

BAD ORIGINAL

durchgeführt, kann aber auch bei erhöhter oder erniedrigter Temperatur durchgeführt werden. Durch Zugabe eines Salzes der Jodwasserstoffsäure oder der Thiocyansäure, z.B. eines Alkalimetall-, wie Natriumsalzes, kann die Reaktion beschleunigt werden.

In die Reaktion können sowohl optisch inaktive cis- oder trans-Verbindungen der Formel III, als auch Mischungen davon, oder entsprechende optisch aktive Verbindungen eingesetzt werden. Die eintretende Gruppe $R_1-(CH_2)_4-C(=Z')-S-$ wird von der Gruppe $R_3-CH_2-$ bevorzugt in die trans-Stellung dirigiert, unabhängig davon, ob W zur Gruppe $R_3-CH_2-$ in cis- oder trans-Stellung steht. Obwohl die trans-Isomeren überwiegend gebildet werden, können doch gelegentlich auch die cis-Isomeren isoliert werden. Die Auftrennung der cis- und trans-Isomeren erfolgt wie oben beschrieben, nach konventionellen Methoden, insbesondere durch Chromatographie und/oder durch Kristallisation.

Die nachträgliche Ozonisierung einer Methyliden-Gruppe Z' kann wie weiter unten angegeben durchgeführt werden. Ein erhaltenes Racemat der Formel IV kann in die optisch aktiven Verbindungen getrennt werden.

Ein Azetidinon der Formel III, worin $R_3$ und W jeweils Acetoxy bedeuten, ist in der Deutschen Offenlegungsschrift Nr. 29 50 898 beschrieben. Andere Azetidinone der Formel III können nach an sich bekannten Verfahren hergestellt werden, beispielsweise indem man einen Vinylester der Formel $R_3-CH_2-CH=CH-W$ mit Chlorsulfonylisocyanat umsetzt und das entstandene Cycloaddukt mit einem Reduktionsmittel, z.B. Natriumsulfit, umsetzt. Bei dieser Synthese werden gewöhnlich Mischungen von cis- und trans-Isomeren erhalten, die gewünschtenfalls in die reinen cis- oder trans-Isomeren, z.B. durch Chromatographie und/oder Kristallisation oder Destillation, aufgetrennt werden können. Die reinen cis- und trans-Isomeren liegen als Racemate vor und können in ihre optischen

BAD ORIGINAL

Antipoden getrennt werden, beispielsweise wenn Acyl in einem Acyloxyrest W in Verbindungen der Formel III von einer optisch aktiven Säure stammt. Die Verbindungen der Formel III, insbesondere ihre optisch aktiven Vertreter, können auch nach den unten in den Reaktionsschemata II und III angegebenen Verfahren hergestellt werden.

Stufe 1.2: Eine α-Hydroxycarbonsäureverbindung der Formel V wird erhalten, indem man eine Verbindung der Formel IV mit einer Glyoxylsäure-Verbindung der Formel $OHC-C(=O)-R_2^A$ oder einem geeigneten Derivat davon, wie einem Hydrat, Hemihydrat oder Halbacetal, z.B. einem Halbacetal mit einem Niederalkanol, z.B. Methanol oder Aethanol, umsetzt und, wenn gewünscht, in einer erhältlichen Verbindung der Formel V, worin $R_3$ Hydroxy ist, Hydroxy in geschütztes Hydroxy überführt.

Die Verbindung V erhält man üblicherweise als Gemisch der beiden Isomeren (bezüglich der Gruppierung $>CH\sim OH$). Man kann aber auch die reinen Isomeren davon isolieren.

Die Anlagerung der Glyoxylsäureesterverbindung an das Stickstoffatom des Lactamrings findet bei Raumtemperatur oder, falls notwendig, unter Erwärmen, z.B. bis etwa 100°C, und zwar in Abwesenheit eines eigentlichen Kondensationsmittels und/oder ohne Bildung eines Salzes statt. Bei Verwendung des Hydrats der Glyoxylsäureverbindung wird Wasser gebildet, das, wenn notwendig, durch Destillation, z.B. azeotrop, oder durch Verwendung eines geeigneten Dehydrationsmittels, wie eines Molekularsiebs, entfernt wird. Vorzugsweise arbeitet man in Gegenwart eines geeigneten Lösungsmittels, wie z.B. Dioxan, Toluol oder Dimethylformamid, oder Lösungsmittelgemisches, wenn erwünscht oder notwendig, in der Atmosphäre eines Inertgases, wie Stickstoff.

BAD ORIGINAL

In die Reaktion können sowohl reine optisch inaktive cis- oder trans-Verbindungen der Formel IV, als auch Mischungen davon, oder entsprechende optisch aktive Verbindungen eingesetzt werden. Ein erhaltenes Racemat der Formel V kann in die optisch aktiven Verbindungen getrennt werden.

Stufe 1.3: Verbindungen der Formel VI, worin $X_o$ für eine reaktionsfähige veresterte Hydroxygruppe, insbesondere für Halogen oder organisches Sulfonyloxy steht, werden hergestellt, indem man in einer Verbindung der Formel V die sekundäre Hydroxygruppe in eine reaktionsfähige veresterte Hydroxygruppe, insbesondere in Halogen, z.B. Chlor oder Brom, oder in eine organische Sulfonyloxygruppe, wie Niederalkansulfonyloxy, z.B. Methansulfonyloxy, oder Arensulfonyloxy, z.B. Benzol- oder 4-Methylbenzolsulfonyloxy, umwandelt.

In den Ausgangsverbindungen der Formel V steht $R_3$ vorzugsweise für eine geschützte Hydroxygruppe.

Die Verbindungen der Formel VI kann man in Form von Gemischen der Isomeren (bezüglich der Gruppierung $>CH\sim X_o$) oder in Form von reinen Isomeren erhalten.

Die obige Reaktion wird durch Behandeln mit einem geeigneten Veresterungsmittel durchgeführt, z.B. mit einem Thionylhalogenid, z.B. -chlorid, einem Phosphoroxyhalogenid, besonders -chlorid, einem Halogenphosphoniumhalogenid, wie Triphenylphosphondibromid oder -dijodid, oder einem geeigneten organischen Sulfonsäurehalogenid, wie -chlorid, vorzugsweise in Gegenwart eines basischen, in erster Linie eines organischen basischen Mittels, wie eines aliphatischen tertiären Amins, z.B. Triäthylamin, Diisopropyläthylamin oder "Polystyrol-Hünigbase", oder einer heterocyclischen Base vom Pyridintyp, z.B. Pyridin oder Collidin. Vorzugsweise arbeitet man in Gegenwart eines geeigneten Lösungsmittels, z.B. Dioxan oder Tetrahydrofuran, oder eines Lösungsmittelgemisches, wenn notwendig, unter Kühlen und/oder in der Atmosphäre eines Inertgases, wie Stickstoff.

In einer so erhältlichen Verbindung der Formel VI kann eine reaktionsfähige veresterte Hydroxygruppe $X_o$ in an sich bekannter Weise in eine andere reaktionsfähige veresterte Hydroxygruppe umgewandelt werden. So kann man z.B. ein Chloratom durch Behandeln der entsprechenden Chlorverbindung mit einem geeigneten Bromid- oder Jodidsalz, wie Lithiumbromid oder -jodid, vorzugsweise in Gegenwart eines geeigneten Lösungsmittels, wie Aether, durch ein Brom- bzw. Jodatom austauschen.

In die Reaktion können sowohl reine optische inaktive cis- oder trans-Verbindungen der Formel V als auch Mischungen davon, oder entsprechende optisch aktive Verbindungen eingesetzt werden. Ein erhaltenes Racemat der Formel VI kann in die optisch aktiven Verbindungen getrennt werden.

Stufe 1.4: Das Ausgangsmaterial der Formel II' wird erhalten, indem man eine Verbindung der Formel VI, worin $X_o$ für eine reaktionsfähige veresterte Hydroxygruppe steht, mit einer geeigneten Phosphin-Verbindung, wie einem Triniederalkylphosphin, z.B. Tri-n-butyl-phosphin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt, wobei man je nach Wahl des Reagenzes eine Verbindung der Formel II (bzw. II') oder IIa erhalten kann.

Die obige Reaktion wird vorzugsweise in Gegenwart eines geeigneten inerten Lösungsmittels, wie eines Kohlenwasserstoffs, z.B. Hexan, Cyclohexan, Benzol, Toluol oder Xylol, oder eines Aethers, z.B. Dioxan, Tetrahydrofuran oder Diäthylenglykol-dimethyläther, oder

BAD ORIGINAL

eines Lösungsmittelgemisches vorgenommen. Je nach Reaktionsfähigkeit arbeitet man unter Kühlen oder bei erhöhter Temperatur, etwa zwischen -10° und +100°, bevorzugt bei etwa 20° bis 80°, und/oder in der Atmosphäre eines inerten Gases, wie Stickstoff. Zur Verhinderung oxidativer Prozesse können katalytische Mengen eines Antioxidans, z.B. Hydrochinon, zugesetzt werden.

Dabei arbeitet man bei der Verwendung einer Phosphinverbindung üblicherweise in Gegenwart eines basischen Mittels, wie einer organischen Base, z.B. eines Amins, wie Triäthylamin, Diisopropyl-äthyl-amin oder "Polystyrol-Hünigbase", und gelangt so direkt zum Ylid-Ausgangsmaterial der Formel II (bzw. II'), das aus dem entsprechenden Phosphoniumsalz gebildet wird.

In die Reaktion können sowohl reine, optisch inaktive cis- oder trans-Verbindungen der Formel VI, als auch Mischungen davon, oder entsprechende optisch aktive Verbindungen eingesetzt werden. Ein erhaltenes Racemat der Formel II kann in die optisch aktiven Verbindungen getrennt werden.

Die Ylide der Formel II', worin Z' Sauerstoff oder Schwefel ist, können direkt in die Cyclisierungsreaktion zur Herstellung der Endprodukte der Formel I eingesetzt werden. Man kann aber auch in Verbindungen der Formel II', worin $R_3$ eine geschützte Hydroxygruppe, z.B. eine hydrolytisch leicht spaltbare geschützte Hydroxygruppe, wie trisubstituiertes Silyloxy, ist, zuerst die Hydroxyschutzgruppe abspalten und dann die erhaltene Verbindung der Formel II', worin $R_3$ Hydroxy ist, in die Cyclisierungsreaktion einsetzen. Weiterhin kann man in Verbindungen der Formel II', worin $R_1$ geschütztes Amino ist, die Aminoschutzgruppe abspalten.

In den Verbindungen II', IV, V und VI kann eine gegebenenfalls substi-

tuierte Methylidengruppe Z' durch Ozonisierung und anschliessender Reduktion des gebildeten Ozonids, gemäss dem nachfolgend in der Stufe 3.3 beschriebenen Verfahren, in die Oxogruppe Z überführt werden.

Ausgangsverbindungen der Formel III, worin W ein Sulfonylrest $HO-A-SO_2-$ ist, können auch nach dem folgenden Reaktionsschema II hergestellt werden.

Reaktionsschema II

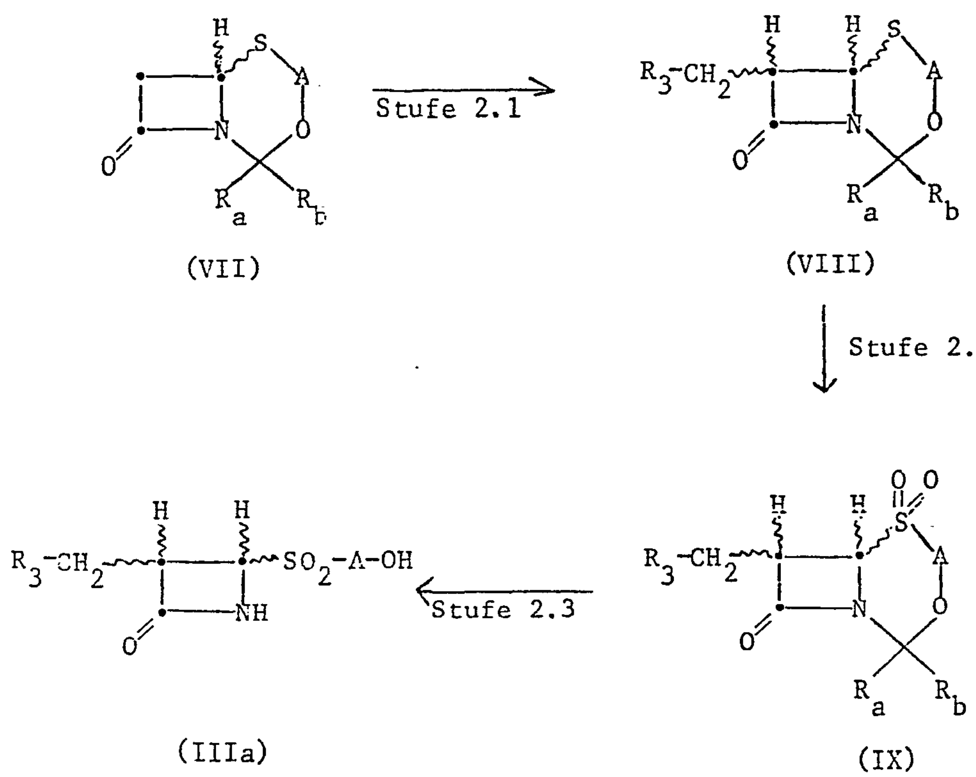


(VII) (VIII) (IX) (IIIa)

In den Verbindungen der Formel (VII)-(IX) und (IIIa) stellt A einen Niederalkylenrest mit 2 bis 3 Kohlenstoffatomen zwischen den beiden Heteroatomen dar und ist in erster Linie Aethylen oder 1,2-Propylen, kann jedoch auch 1,3-Propylen, 1,2-, 2,3- oder 1,3-Butylen sein.

In den Verbindungen der Formel (VII) - (IX) steht jeder der Reste $R_a$ und $R_b$ für Wasserstoff oder für einen über ein Kohlenstoffatom mit dem

Ringkohlenstoffatom verbundenen organischen Rest, wobei die beiden Reste $R_a$ und $R_b$ untereinander verknüpft sein können, in erster Linie für Wasserstoff, Niederalkyl, z.B. Methyl, Aethyl, n-Propyl oder Isopropyl, gegebenenfalls substituiertes Phenyl, oder Phenylniederalkyl, z.B. Benzyl, oder, wenn zusammengenommen, Niederalkylen vorzugsweise mit 4 bis 6 Kohlenstoffatomen, z.B. 1,4-Butylen oder 1,5-Pentylen.

In den Verbindungen der Formel (VIII), (IX) und (IIIa) ist der Rest $R_3$ Hydroxy oder vorzugsweise eine der genannten geschützten Hydroxygruppen, z.B. gegebenenfalls substituiertes 1-Phenylniederalkoxy, gegebenenfalls substituiertes Phenylniederalkoxycarbonyloxy oder trisubstituiertes Silyloxy.

Stufe 2.1

Eine Verbindung der Formel (VIII) wird erhalten, indem man eine bicyclische Verbindung der Formel (VII) mit einem Metallierungreagenz und einem den Rest $R_3$-$CH_2$- einführenden Elektrophil umsetzt, anschliessend das entstandene Produkt mit einer Protonenquelle behandelt und gewünschtenfalls eine erhältliche Verbindung der Formel VIII, worin $R_3$ Hydroxy ist, in eine Verbindung der Formel VIII überführt, worin $R_3$ geschütztes Hydroxy bedeutet.

Geeignete Metallierungsreagenzien sind z.B. substituierte und unsubstituierte Alkalimetallamide, Alkalimetallhydride oder Alkalimetallniederalkylverbindungen, worin das Alkalimetall z.B. Natrium oder insbesondere Lithium ist, z.B. Natrium- oder Lithiumamid, Lithium-bistrimethylsilylamid, Natriumhydrid, Lithiumhydrid und bevorzugt Lithiumdiisopropylamid und Butyllithium.

Ein den Rest $R_3$-$CH_2$- einführendes Elektrophil ist beispielsweise Formaldehyd, wobei Verbindungen der Formel (VIII) gebildet werden, worin $R_3$ Wasserstoff ist, oder ein funktionelles Derivat von Formaldehyd der Formel $R_3$-$CH_2$-X, worin X eine nucleofuge Abgangsgruppe, insbesondere Halogen, z.B. Chlor, Brom oder Jod,oder Sulfonyloxy, z.B.

Methansulfonyloxy oder 4-Toluolsulfonyloxy, darstellt. Bevorzugte den Rest $R_3$-$CH_2$- einführende Elektrophile sind Formaldehyd und gegebenenfalls substituiertes Benzyloxymethylchlorid.

Für die Metallierungsreaktion geeignete Lösungsmittel dürfen keinen aktiven Wasserstoff enrhalten und sind z.B. Kohlenwasserstoffe, z.B. Hexan, Benzol, Toluol oder Xylol, Aether, z.B. Diäthyläther, Tetrahydrofuran oder Dioxan, oder Säureamide, z.B. Hexamethylphosphorsäuretriamid.

Das metallierte Zwischenprodukt braucht nicht isoliert, sondern kann im Anschluss an die Metallierungsreaktion mit einem den Rest $R_3$-$CH_2$-einführenden Elektrophil umgesetzt werden. Die Metallierungsreaktion erfolgt bei Temperaturen von etwa -100°C bis etwa Raumtemperatur, vorzugsweise unterhalb -30°C, vorzugsweise in einer Inertgas-, wie Stickstoffatmosphäre. Die weitere Umsetzung kann unter den gleichen Bedingungen erfolgen. Dabei wird Formaldehyd vorzugsweise in gasförmiger, monomerer Form in das Reaktionsgemisch eingeleitet. Monomerer Formaldehyd ist beispielsweise durch thermische Depolymerisation von Paraformaldehyd oder durch thermische Zersetzung von Formaldehydcyclohexylhemiacetal erhältlich.

Für die Metallierungsreaktion können sowohl die Antipoden von Verbindungen der Formel (VII) als auch deren racemische oder diastereomere Mischungen eingesetzt werden.

Der Angriff des den Rest $R_3$-$CH_2$- einführenden Elektrophils an das Substrat erfolgt im allgemeinen stereospezifisch. Wird als Ausgangsmaterial ein Azetidinon der Formel (VII) mit R-Konfiguration am Kohlenstoffatom 4 des Azetidinon-Rings benutzt, so entsteht überwiegend eine Verbindung der Formel (VIII) mit R-Konfiguration am Kohlenstoffatom 4 und S-Konfiguration am Kohlenstoffatom 3 des Azetidinon-Rings, d.h., der Angriff des Elektrophils erfolgt überwiegend trans-ständig.

Nach der Umsetzung wird das Reaktionsprodukt mit einer Protonenquelle behandelt, z.B. mit Wasser, einem Alkohol, wie Methanol oder Aethanol, einer organischen oder anorganischen Säure, z.B. Essigsäure, Salzsäure, Schwefelsäure, oder einer ähnlichen Protonen-abgebenden Verbindung, bevorzugt wiederum bei niederen Temperaturen.

In einer erhaltenen Verbindung der Formel (VIII), worin $R_3$ Wasserstoff ist, kann man die Hydroxygruppe in an sich bekannter Weise, z.B. durch Veräthern oder Verestern, insbesondere wie oben beschrieben, schützen.

Ausgangsverbindungen der Formel (VII) können beispielsweise erhalten werden, indem man ein Acetat der Formel

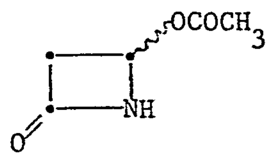

(X).

mit einer Mercaptoverbindung der Formel HS-A-OH umsetzt und die entstandene Thioverbindung der Formel

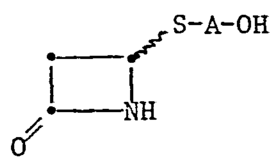

(Xa) ,

wie in der Europäischen Patentanmeldung Nr. 23887 beschrieben, mit einer Carbonylverbindung der Formel $(R_a,R_b)C=O$ behandelt.

Bei der Umsetzung von Acetaten der Formel (X) mit Mercaptanen der Formel HS-A-OH, die in A ein Chiralitätszentrum besitzen, z.B. Antipoden eines 2-Mercapto-1-propanols, wie (2R)-2-Mercapto-1-propanol, entstehen Diastereomerengemische von Verbindungen der Formel (Xa), die sich durch übliche Methoden, beispielsweise wie oben beschrieben, in die einzelnen Antipoden auftrennen lassen. Die individuellen Antipoden, z.B. das (4R)-Antipode der Formel

BAD ORIGINAL

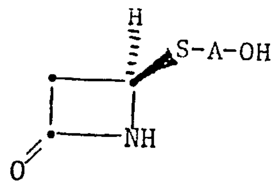

(Xb),

lassen sich ohne Konfigurationsänderung zu den entsprechenden Antipoden der in den Reaktionsschemata I und II genannten Zwischenprodukte (II) bis (VI), (VIII) und (IX) und der Endprodukte der Formel (I) weiterverarbeiten. Da, wie oben beschrieben, die Einführung des gegebenenfalls geschützten Hydroxymethyl-Substituenten in den Azetidinon-Ring der Verbindung der Formel (VII) stereospezifisch (trans) erfolgt, sind ausgehend von Verbindungen der Formel (Xb) Zwischenprodukte der Formel (II) bis (VI), (VIII) und (IX) und Endprodukte der Formel (I) zugänglich, in denen der Azetidinon-Ring in 4-Stellung die R- und in 3-Stellung die S-Konfiguration einnimmt.

Stufe 2.2

Ein Sulfon der Formel IX kann hergestellt werden, indem man die Thioverbindung der Formel III mit einem Oxidationsmittel behandelt und gewünschtenfalls eine verfahrensgemäss erhältliche Verbindung der Formel IX, worin $R_3$ Hydroxy ist, in eine Verbindung der Formel IX überführt, worin $R_3$ eine geschützte Hydroxygruppe ist.

Geeignete Oxidationsmittel sind beispielsweise Wasserstoffperoxid, organische Persäuren, insbesondere aliphatische oder aromatische Percarbonsäuren, z.B. Peressigsäure, Perbenzoesäure, Chlorperbenzoesäure, z.B. 3-Chlorperbenzoesäure, oder Monoperphthalsäure, oxidierende anorganische Säuren und deren Salze, z.B. Salpetersäure, Chromsäure, Kaliumpermanganat, oder Alkalimetallhypochlorite, z.B. Natriumhypochlorit. Die Ueberführung kann aber auch durch anodische Oxidation erfolgen.

Die Oxidation wird bevorzugt in einem geeigneten inerten Lösungsmittel, beispielsweise einem Halogenkohlenwasserstoff, z.B. Methylenchlorid, Chloroform oder Tetrachlorkohlenstoff, einem Alkohol, z.B. Methanol oder Aethanol, einem Keton, z.B. Aceton, einem Aether, Diäthyläther, Dioxan oder Tetrahydrofuran, einem Amid, z.B. Dimethylformamid, einem Sulfon, z.B. Dimethylsulfon, einer flüssigen organischen Carbonsäure, z.B. Essigsäure, oder in Wasser oder einem Gemisch dieser Lösungsmittel, insbesondere einem wasserhaltigen Gemisch, z.B. wässriger Essigsäure, bei Raumtemperatur, oder unter Kühlen oder leichtem Erwärmen, d.h. bei etwa -20°C bis etwa +90°C, bevorzugt bei etwa Raumtemperatur durchgeführt. Die Oxidation kann auch stufenweise durchgeführt werden, indem zunächst bei niederer Temperatur, d.h. bei etwa -20°C bis etwa 0°C, bis zum Sulfoxid oxidiert wird, das gegebenenfalls isoliert wird, worauf in einem zweiten Schritt, bevorzugt bei höherer Temperatur, etwa bei Raumtemperatur, das Sulfoxid zum Sulfon der Formel (IX) oxidiert wird.

Zur Aufarbeitung kann gegebenenfalls noch vorhandenes überschüssiges Oxidationsmittel durch Reduktion, insbesondere durch Behandeln mit einem Reduktionsmittel, wie einem Thiosulfat, z.B. Natriumthiosulfat, zerstört werden.

In die Reaktion können sowohl optisch inaktive Verbindungen der Formel (VIII) als auch entsprechende optisch aktive Verbindungen, insbesondere solche mit 3S,4R-Konfiguration im Azetidinon-Ring, eingesetzt werden.

Stufe 2.3

Verbindungen der Formel (IIIa) können hergestellt werden, indem man ein bicyclisches Amid der Formel (IX) mit einem geeigneten Solvolysereagenz solvolysiert und, wenn gewünscht, in einer verfahrensgemäss erhältlichen Verbindung der Formel (IIIa) eine freie Hydroxygruppe $R_3$

in eine geschützte Hydroxygruppe $R_3$ umwandelt.

Geeignete Solvolysereagenzien sind beispielsweise organische Säuren, z.B. Niederalkancarbonsäuren, wie Ameisensäure oder Essigsäure, oder Sulfonsäuren, z.B. 4-Toluolsulfonsäure oder Methansulfonsäure, Mineralsäuren, z.B. Schwefel- oder Salzsäure, ferner Niederalkanole, z.B. Methanol oder Aethanol, oder Niederalkandiole, z.B. Aethylenglykol.

Die genannten Solvolysereagenzien werden unverdünnt oder mit Wasser verdünnt zugegeben. Die Solvolyse kann auch mit reinem Wasser durchgeführt werden. Die Solvolyse mit dem sauren Reagenz findet bevorzugt in wässriger Lösung dieses Reagenzes und bei Temperaturen von etwa -20°C bis etwa 150°C, bevorzugt bei Zimmertemperatur bis 110°C statt.

In die Reaktion können sowohl optisch inaktive Verbindungen der Formel (IX), z.B. Racemate oder Diastereomerengemische, als auch entsprechende optisch aktive Verbindungen, insbesondere solche mit 3S,4R-Konfiguration im Azetidinon-Ring, eingesetzt werden.

Erhaltene Isomerengemische von Verbindungen der Formel (VIII), (IX) und (IIIa), wie Racemate oder Diastereomerengemische, können in an sich bekannter Weise, z.B. wie oben beschrieben, in die einzelnen Isomeren, wie Antipoden, getrennt werden.

Erfindungsgemäss verwendbare optisch aktive trans-Verbindungen der Formel (III) können auch nach dem folgenden Reaktionsschema III hergestellt werden:

Reaktionsschema III

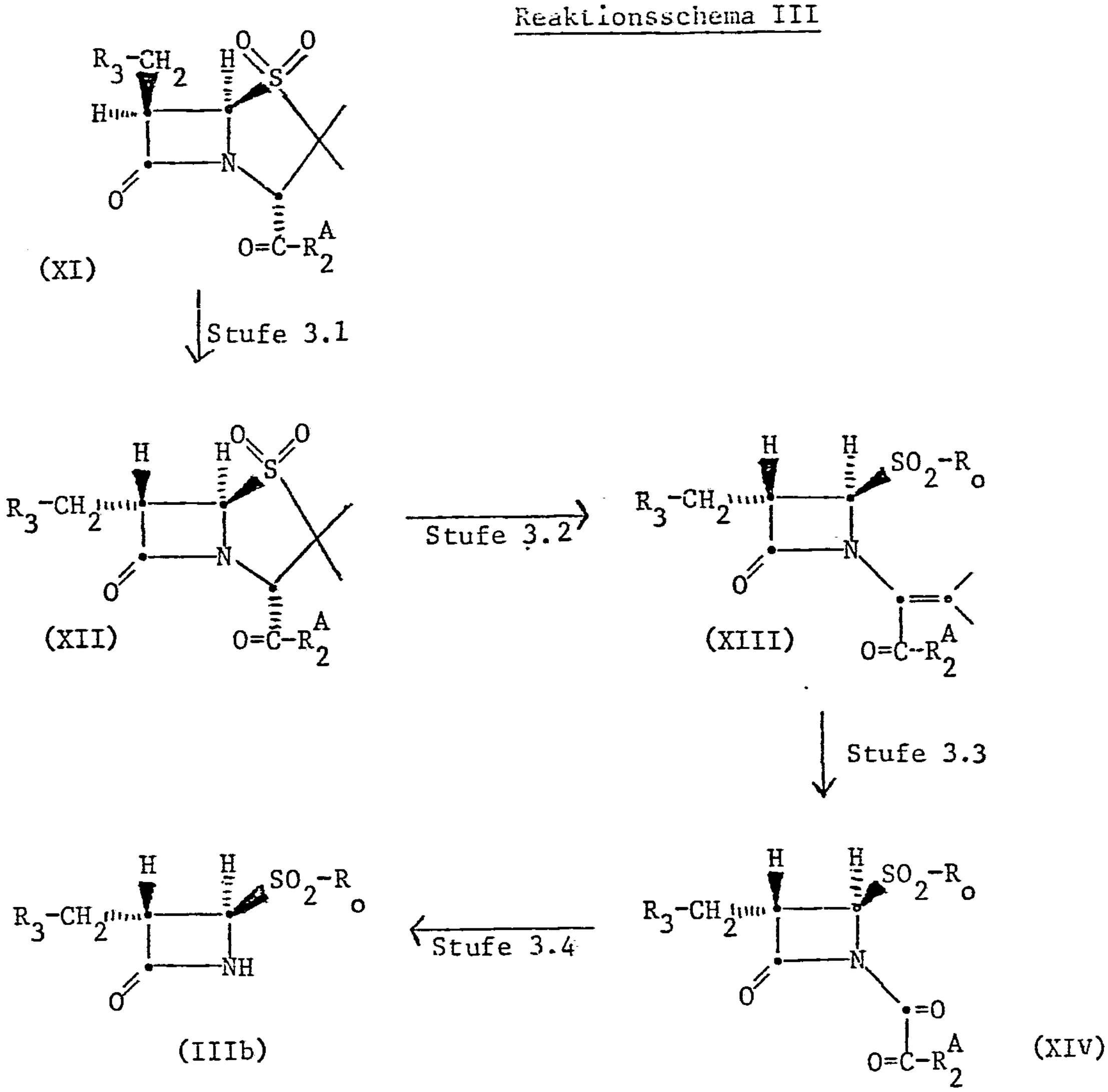


(XI) (XII) (XIII) (XIV) (IIIb)

In den Verbindungen der Formel (XI) - (XIV) und (IIIb) steht $R_3$ für Hydroxy oder insbesondere für eine geschützte Hydroxygruppe.

Stufe 3.1

Verbindungen der Formel (XII) sind bekannt oder können auf an sich bekannte Weise hergestellt werden. Sie können auch nach einem neuen Verfahren hergestellt werden, indem man eine Verbindung der Formel (XI) epimerisiert und, wenn gewünscht, in einer verfahrensgemäss erhältlichen Verbindung der Formel (XII) eine geschützte Hydroxygruppe $R_3$ in eine andere geschützte Hydroxygruppe $R_3$ überführt.

ORIGINAL

Die Epimerisierung erfolgt beispielsweise in Gegenwart eines basischen Mittels, wie eines Amins, z.B. eines Triniederalkylamins, z.B. Triäthylamin oder Aethyl-diisopropylamin, eines tertiären Amins, z.B. N,N-Dimethylanilin, eines aromatischen Amins, z.B. Pyridin, oder eines bicyclischen Amins, z.B. 1,5-Diazabicyclo[5,4,0]undec-5-en oder 1,5-Diazabicyclo[4,3,0]non-5-en, oder eines Alkalimetallniederalkanolats, z.B. Natriummethanolat, Natriumäthanolat oder Kalium-tert.-butanolat, in einem inerten Lösungsmittel, beispielsweise einem Aether, z.B. Diäthyläther, Dimethoxyäthan, Tetrahydrofuran oder Dioxan, Acetonitril oder Dimethylformamid, gegebenenfalls bei etwas erhöhter oder erniedrigter Temperatur, z.B. bei 0°C bis 50°C, vorzugsweise bei Raumtemperatur.

In den verfahrensgemäss erhältlichen Verbindungen der Formel (XII) kann eine geschützte Hydroxygruppe $R_3$ durch eine andere geschützte Hydroxygruppe $R_3$, beispielsweise eine hydrogenolytisch spaltbare geschützte Hydroxygruppe durch eine solvolytisch spaltbare geschützte Hydroxygruppe, ersetzt werden. Hydroxyschutzgruppen sind insbesondere die oben genannten, hydrogenolytisch abspaltbare Schutzgruppen beispielsweise wie angegeben substituiertes 1-Phenylniederalkyl oder Phenylniederalkoxycarbonyl, solvolytisch abspaltbare Schutzgruppen beispielsweise wie angegeben trisubstituiertes Silyl.

Die Umsetzung kann so durchgeführt werden, dass man zunächst die hydrogenolytisch abspaltbare Hydroxyschutzgruppe entfernt und in die entstandene Verbindung der Formel XII, worin $R_3$ Hydroxy ist, eine solvolytisch abspaltbare Hydroxyschutzgruppe einführt.

Die Abspaltung der hydrogenolytisch abspaltbaren Schutzgruppe erfolgt z.B. mit Wasserstoff oder einem Wasserstoffdonator, z.B. Cyclohexen oder Cyclohexadien, in Gegenwart eines

Hydrierungskatalysators, wie eines Palladiumkatalysators, z.B. Palladium auf Kohle, in einem inerten Lösungsmittel, wie einem halogenierten Kohlenwasserstoff, z.B. Methylenchlorid, einem Niederalkanol, z.B. Methanol oder Aethanol, einem Aether, z.B. Dioxan oder Tetrahydrofuran, oder auch in Wasser oder in Gemischen davon, bei einer Temperatur von etwa 0° bis etwa 80°C, vorzugsweise bei Raumtemperatur.
Die Abspaltung kann auch mit einem reduzierenden Metall, wie Zink, oder einer reduzierenden Metallegierung, z.B. Kupfer-Zink-Legierung, in Gegenwart eines Protonen-abgebenden Mittels, wie einer organischen Säure, z.B. Essigsäure, oder auch eines Niederalkanols, z.B. Aethanol, durchgeführt werden.

Die Einführung der solvolytisch abspaltbaren Hydroxyschutzgruppe erfolgt beispielsweise mit einer Verbindung der Formel $R_3'-X_3$, worin $R_3'$ die Hydroxyschutzgruppe und $X_3$ z.B. eine reaktionsfähige veresterte Hydroxygruppe, beispielsweise Halogen, z.B. Chlor, Brom oder Jod, oder Sulfonyloxy, wie Methansulfonyloxy, Benzolsulfonyloxy oder 4-Toluolsulfonyloxy, bedeutet.

Die Umsetzung erfolgt in einem inerten Lösungsmittel, wie einem Aether, z.B. Diäthyläther, Dioxan oder Tetrahydrofuran, einem Kohlenwasserstoff, z.B. Benzol oder Toluol, einem Halogenkohlenwasserstoff, z.B. Methylenchlorid, in Dimethylsulfoxid oder Acetonitril, in Gegenwart eines basischen Kondensationsmittels, wie eines Alkalimetallhydroxids oder -carbonats, z.B. Natrium- oder Kaliumhydroxid oder Natrium- oder Kaliumcarbonat, eines Alkalimetallamids oder -hydrids, z.B. Natriumamid oder Natriumhydrid, eines Alkalimetallniederalkanolats, z.B. Natriummethanolat oder -ethanolat oder Kalium-tert.-butanolat, oder eines Amins, z.B. Triäthylamin, Pyridin oder Imidazol, bei Raumtemperatur oder erhöhter oder erniedrigter Temperatur, z.B. bei etwa -20°C bis etwa 80°C, bevorzugt bei Raumtemperatur.

BAD ORIGINAL

Ausgangsverbindungen der Formel (XI) sind beispielsweise aus der Deutschen Offenlegungsschrift 3 039 504 und aus der britischen Patentanmeldung 20 61 930 bekannt.

Stufe 3.2

Eine Verbindung der Formel (XIII) kann hergestellt werden, indem man eine Penam-Verbindung der Formel (XII) mit einem basischen Mittel und mit einem den Rest $R_o$ einführenden Veresterungsmittel behandelt.

Ein geeignetes basisches Mittel ist beispielsweise eines der unter Stufe 3.1 genannten basischen Mittel, insbesondere eines der genannten bicyclischen Amine, ferner auch ein Alkalimetallamid oder -hydrid, z.B. Natriumamid oder Natriumhydrid.

Ein Rest $R_o$ ist beispielsweise einer der unter Stufe 1.1 genannten organischen Reste, insbesondere gegebenenfalls substituiertes Niederalkyl, z.B. Methyl, Aethyl oder 2-Hydroxyäthyl, oder Benzyl.

Ein den Rest $R_o$ einführendes Veresterungsmittel ist z.B. eine Verbindung der Formel $R_o$-$X_4$, worin $X_4$ reaktionsfähiges verestertes Hydroxy, z.B. Halogen, wie Chlor, Brom oder Jod, oder Sulfonyloxy, wie Methansulfonyloxy, Benzolsulfonyloxy oder 4-Toluolsulfonyloxy, bedeutet. Zur Einführung eines 2-Hydroxyäthylrestes ist auch Aethylenoxid geeignet.

Die Umsetzung wird vorzugsweise zweistufig durchgeführt, wobei man in der ersten Stufe die Penam-Verbindung der Formel (XIII) mit mindestens äquimolekularen Mengen des basischen Mittels behandelt und ein erhältliches Zwischenprodukt der Formel

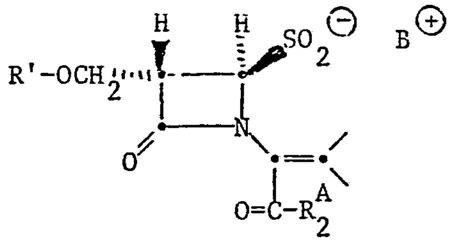

(XIIIa)

worin $B^{\oplus}$ die protonierte Form (Kation) des basischen Mittels darstellt, vorzugsweise ohne Isolierung aus dem Reaktionsgemisch mit dem Veresterungsmittel umsetzt. Die Reaktion wird in einem inerten Lösungsmittel, beispielsweise einem Aether, z.B. Diäthyläther, Dimethoxyäthan, Tetrahydrofuran oder Dioxan, in Acetonitril, Dimethylformamid oder Hexamethylphosphorsäuretriamid, gegebenenfalls bei etwas erhöhter oder erniedrigter Temperatur, z.B. bei etwa 0°C bis 50°C, vorzugsweise bei Raumtemperatur, durchgeführt. In einer bevorzugten Ausführungsform des Verfahrens wird die Penam-Verbindung der Formel (XII) in situ hergestellt, indem man wie unter Stufe 3.1 beschrieben, eine Verbindung der Formel (XI) zuerst mit katalytischen Mengen des basischen Mittels, z.B. 1,5-Diazabicyclo[5,4,0]undec-5-en, behandelt, und dann mit zumindest äquimolaren Mengen des gleichen basischen Mittels und des Veresterungsmittels weiter zu den Verbindungen der Formel (XIII) umsetzt.

<u>Stufe 3.3</u>

Ein Oxalyl-azetidinon der Formel (XIV) kann hergestellt werden, indem man eine Verbindung der Formel (XIII) ozonisiert und das gebildete Ozonid reduktiv zur Oxo-Verbindung spaltet.

Die Ozonisierung wird üblicherweise mit einem Ozon-Sauerstoff-Gemisch in einem inerten Lösungsmittel, wie einem Niederalkanol, z.B. Methanol oder Aethanol, einem Niederalkanon, z.B. Aceton, einem gegebenenfalls halogenierten Kohlenwasserstoff, z.B. einem Halogenniederalkan, wie Methylenchlorid oder Tetrachlorkohlenstoff, oder in einem Lösungsmittelgemisch, inkl. einem wässrigen Gemisch, vorzugsweise unter

ORIGINAL

Kühlen, z.B. bei Temperaturen von etwa -80° bis etwa 0°C, durchgeführt.

Ein als Zwischenprodukt erhaltenes Ozonid wird, üblicherweise ohne isoliert zu werden, reduktiv zu einer Verbindung der Formel XIV gespalten, wobei man katalytisch aktivierten Wasserstoff, z.B. Wasserstoff in Gegenwart eines Schwermetallhydrierungskatalysators, wie eines Nickel-, ferner Palladiumkatalysators, vorzugsweise auf einem geeigneten Trägermaterial, wie Calciumcarbonat oder Kohle, oder chemische Reduktionsmittel, wie reduzierende Schwermetalle, inkl. Schwermetallegierungen oder -amalgame, z.B. Zink, in Gegenwart eines Wasserstoffdonators, wie einer Säure, z.B. Essigsäure, oder eines Alkohols, z.B. Niederalkanols, reduzierende anorganische Salze, wie Alkalimetalljodide, z.B. Natriumjodid, oder Alkalimetallhydrogensulfite, z.B. Natriumhydrogensulfit, in Gegenwart eines Wasserstoffdonators, wie einer Säure, z.B. Essigsäure, oder Wasser, oder reduzierende organische Verbindungen, wie Ameisensäure, verwendet. Als Reduktionsmittel können auch Verbindungen zum Einsatz kommen, die leicht in entsprechende Epoxidverbindungen oder Oxide umgewandelt werden können, wobei die Epoxidbildung aufgrund einer C,C-Doppelbindung und die Oxidbildung aufgrund eines vorhandenen Oxid-bildenden Hetero-, wie Schwefel-, Phosphor- oder Stickstoffatoms erfolgen kann. Solche Verbindungen sind z.B. geeignet substituierte Aethenverbindungen (die in der Reaktion in Aethylenoxidverbindungen umgewandelt werden), wie Tetracyanäthylen, oder insbesondere geeignete Sulfidverbindungen (die in der Reaktion in Sulfoxidverbindungen umgewandelt werden), wie Diniederalkylsulfide, in erster Linie Dimethylsulfid, geeignete organische Phosphorverbindungen, wie ein gegebenenfalls durch Phenyl und/oder Niederalkyl, z.B. Methyl, Aethyl, n-Propyl oder n-Butyl, substituiertes Phosphin (das in der Reaktion in ein Phosphinoxid umgewandelt wird), wie Triniederalkyl-phosphine, z.B. Tri-n-butylphosphin, oder Triphenylphosphin, ferner Triniederalkylphosphite (die in der Reaktion in Phosphorsäuretriniederalkylester

BAD ORIGINAL

übergeführt werden), üblicherweise in der Form von entsprechenden Alkoholadduktverbindungen, wie Trimethylphosphit, oder Phosphorigsäure-triamide, welche gegebenenfalls Niederalkyl als Substituenten enthalten, wie Hexaniederalkyl-phosphorigsäuretriamide, z.B. Hexamethylphosphorigsäuretriamid, letzteres vorzugsweise in der Form eines Methanoladdukts, ferner geeignete Stickstoffbasen (die in der Reaktion in die entsprechenden N-Oxide umgewandelt werden), wie heterocyclische Stickstoffbasen aromatischen Charakters, z.B. Basen vom Pyridintyp und insbesondere Pyridin selber. Die Spaltung des üblicherweise nicht isolierten Ozonids erfolgt normalerweise unter den Bedingungen, die man zu seiner Herstellung anwendet, d.h. in Gegenwart eines geeigneten Lösungsmittels oder Lösungsmittelgemisches, sowie unter Kühlen oder leichtem Erwärmen, wobei man vorzugsweise bei Temperaturen von etwa -10°C bis etwa +25°C arbeitet und die Reaktion üblicherweise bei Raumtemperatur abschliesst.

Stufe 3.4

Ein Azetidinon der Formel (IIIb) kann hergestellt werden, indem man ein Oxalyl-azetidinon der Formel (XIV) solvolysiert.

Die Solvolyse kann als Hydrolyse, als Alkoholyse oder auch als Hydrazinolyse durchgeführt werden. Die Hydrolyse wird mit Wasser, gegebenenfalls in einem mit Wasser mischbaren Lösungsmittel, durchgeführt. Die Alkoholyse wird üblicherweise mit einem Niederalkanol, z.B. Methanol oder Aethanol, vorzugsweise in Gegenwart von Wasser und eines organischen Lösungsmittels, wie eines Niederalkancarbonsäure-niederalkylesters, z.B. Essigsäureäthylester, vorzugsweise bei Raumtemperatur, wenn notwendig unter Kühlen oder Erwärmen, z.B. bei einer Temperatur von etwa 0° bis etwa 80°C, durchgeführt. Die Hydrazinolyse wird auf konventionelle Weise mit einem substituierten Hydrazin, z.B. mit Phenyl- oder einem Nitrophenylhydrazin, wie 2-Nitrophenylhydrazin, 4-Nitrophenylhydrazin oder 2,4-Dinitrophenylhydrazin, das bevorzugt

BAD ORIGINAL

in etwa äquimolarer Menge eingesetzt wird, in einem organischen Lösungsmittel, wie einem Aether, z.B. Tetrahydrofuran, Dioxan, Diäthyläther, einem aromatischen Kohlenwasserstoff, wie Benzol oder Toluol, einem halogenierten Kohlenwasserstoff, wie Methylenchlorid, Chlorbenzol oder Dichlorbenzol, einem Ester wie Aethylacetat, und dergleichen, bei Temperaturen zwischen etwa Raumtemperatur und etwa 65°C durchgeführt.

In einer bevorzugten Ausführungsform des Verfahrens geht man von einer Verbindung der Formel (XIII) aus, die wie angegeben ozonisiert und dann reduktiv zum Oxalyl-azetidinon der Formel (XIV) gespalten wird, welches ohne Isolierung aus dem Reaktionsgemisch weiter zum Azetidinon der Formel (IIIb) umgesetzt wird.

Bei der Ozonolyse entstehen gegebenenfalls geringe Mengen an Säure, welche die Abspaltung eines solvolytisch leicht abspaltbaren Restes $R_3$, z.B. eines trisubstituierten Silyl-Restes, bewirken können. Die dabei entstehende Verbindung der Formel

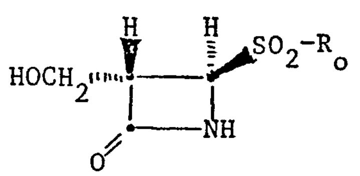

(IIIb')

kann, beispielsweise chromatographisch, vom geschützten Azetidinon abgetrennt und durch erneute Umsetzung mit dem die Hydroxyschutzgruppe $R'_3$ einführenden Mittel der Formel $R'_3-X_3$ in das Azetidinon der Formel (IIIb) übergeführt werden.

In den Verbindungen der Formel (II), (II'), (V), (VI) und (XII)-(XIV) kann eine Gruppe $R_2^A$ nach an sich bekannten Methoden in eine andere Gruppe $R_2^A$ übergeführt werden, wobei unter Berücksichtigung der gegebenenfalls in diesen Verbindungen enthaltenen weiteren

funktionellen Gruppen die gleichen Methoden zur Anwendung gelangen können, wie zur Umwandlung dieses Substituenten in den Verbindungen der Formel (I) angegeben ist.

Die Erfindung betrifft ebenfalls neue Ausgangsprodukte sowie verfahrengemäss erhältliche neue Zwischenprodukte, wie diejenigen der Formel (II) bis (IV), (VIII), (IX) und (XII) bis (XIV), sowie die angegebenen Verfahren zu ihrer Herstellung.

Vorzugsweise werden solche Ausgangsstoffe verwendet und die Reaktionsbedingungen so gewählt, dass man zu den vorstehend als besonders bevorzugt aufgeführten Verbindungen gelangt.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich zur oralen oder zur parenteralen, d.h. intramuskulären, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Kalziumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Tragacant, Methylcellulose, Natriumcarboxymethyl-

BAD ORIGINAL

cellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparte, die, wenn erwünscht, weitere pharmakologisch wertvolle Stoffe enthalten können, werden in an sich bekannter Weise, z.B. mittels konventionellen Mischungs-, Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffs.

Je nach Art der Infektion und Zustand des infizierten Organismus verwendet man tägliche Dosen von etwa 0,1 g bis etwa 5 g[bei der oralen Applikation von (5R, 6S)-2-(4-Aminobutyl)-6-hydroxymethyl-penem-3-carbonsäure z.B. zwei tägliche Dosen zu etwa 0,25 g bis 1 g]zur Behandlung von Warmblütern (Menschen und Tiere) von etwa 70 kg Gewicht.

Die folgenden Beispiele dienen zur Illustration der Erfindung. Temperaturen werden in Celsiusgraden angegeben.

In den Beispielen werden folgende Abkürzungen verwendet:

DC: Dünnschichtchromatogramm,

IR: Infrarotspektrum,

UV: Ultraviolettspektrum,

Smp.: Schmelzpunkt.

DBU: 1,5-Diazabicyclo[5.4.0]undec-5-en

Beispiel 1: trans-2,2-Dimethyl-8-hydroxymethyl-3-oxa-6-thia-1-azabicyclo[5.2.0]nonan-9-on

Eine 2,0 M-Lösung von n-Butyllithium in n-Hexan (0,11 Mol) wird unter Rühren bei -65° und unter Stickstoff zu einer Lösung von 11,0 g (15,5 ml, 0,11 Mol) Diisopropylamin in 200 ml trockenem Tetrahydrofuran zugetropft. Die Mischung wird 15 Minuten bei -65° gerührt. Innerhalb 15 Minuten wird dann eine Lösung von 18,7 g (0,1 Mol) 2,2-Dimethyl-3-oxa-6-thia-1-azabicyclo[5.2.0]-nonan-9-on in 80 ml trockenem Tetrahydrofuran unter Rühren bei -65° hinzugefügt. Die Mischung wird dann weitere 10 Minuten bei -65° gerührt. Formaldehydgas wird in einem getrennten Kolben durch Aufheizen des Formaldehyd/Cyclohexanol-Adduktes auf 150° erzeugt, welches auf herkömmliche Weise durch Umsetzen einer wässrigen Lösung von Formaldehyd mit Cyclohexanol hergestellt wird. Eine überschüssige Menge an trockenem Formaldehydgas wird dann langsam über die Reaktionsmischung bei -65° gerührt. Die Mischung wird 30 Minuten bei -65° gerührt. Die kalte Reaktionsmischung wird auf eine Mischung von 250 g Eis und 250 g Wasser gegossen. 250 ml Chloroform und 110 ml 2,0 N Salzsäure werden hinzugefügt. Nach dem Schütteln der Mischung wird die organische Schicht abgetrennt. Nach der Zugabe von 60 g Natriumchlorid wird die wässrige Schicht anschliessend zweimal mit Chloroform extrahiert. Die organische Lösung wird über Natriumsulfat getrocknet und filtriert. Das Lösungsmittel wird durch Verdampfung entfernt. Der erhaltene Rückstand ist eine gelbliche ölige Flüssigkeit.

Beispiel 2: (5R,7R,8S)-2,2,5-Trimethyl-8-hydroxymethyl-3-oxa-6-thia-1-azabicyclo[5.2.0]nonan-9-on

Diese Verbindung wird auf analoge Weise zu Beispiel 1 durch Umsetzen des metallierten (5R,7R)-2,2,5-Trimethyl-3-oxa-6-thia-1-azabicyclo-[5.2.0]nonan-9-on mit Formaldehyd erhalten.

Beispiel 3: trans-2,2-Dimethyl-8-(4-nitrobenzyloxycarbonyloxymethyl)-3-oxa-6-thia-1-azabicyclo[5.2.0]nonan-9-on

21,6 g (0,1 Mol) fester Chlorkohlensäure-4-nitrobenzylester werden bei -10° zu einer Lösung von 22 g rohem trans-2,2-Dimethyl-8-hydroxymethyl-3-oxa-6-thia-1-azabicyclo[5.2.0]nonan-9-on in 250 ml Methylenchlorid hinzugefügt. 12,2 g (0,1 Mol) festes 4-Dimethylaminopyridin werden in kleinen Teilen innerhalb 30 Minuten zu der Lösung hinzugefügt. Die Mischung wird 4 Stunden bei 3° bis 5° gerührt. Die kalte Mischung wird mit 200 ml 0,5 N wässriger Chlorwasserstoffsäure und einer wässrigen Natriumchloridlösung gewaschen.

Die organische Phase wird über Natriumsulfat getrocknet und filtriert. Das Lösungsmittel wird durch Verdampfen entfernt. Der erhaltene Rückstand ist ein gelber Schaum. Die Umkristallisierung aus Isopropanol ergibt einen Feststoff. Schmelzpunkt 82°.

Beispiel 4: (5R,7R,8S)-2,2,5-Trimethyl-8-(4-nitrobenzyloxycarbonyloxymethyl)-3-oxa-6-thia-1-azabicyclo[5.2.0]nonan-9-on

Diese Verbindung wird auf analoge Weise zu Beispiel 3 durch Umsetzen von (5R,7R,8S)-2,2,5-Trimethyl-8-hydroxymethyl-3-oxa-6-thia-1-azabicyclo[5.2.0] nonan-9-on mit Chlorkohlensäure-4-nitrobenzylester in Gegenwart von 4-Dimethylaminopyridin erhalten.

Beispiel 5: trans-2,2-Dimethyl-8-(4-nitrobenzyloxycarbonyloxymethyl)-3-oxa-6-thia-1-azabicyclo[5.2.0]nonan-9-on-6-dioxid

47,8 g (0,25 Mol) m-Chlorperbenzoesäure werden innerhalb 30 Minuten bei -10° in kleinen Teilen zu einer Lösung von ca. 40 g trans-2,2-Dimethyl-8-(4-nitrobenzyloxycarbonyloxymethyl)-3-oxa-6-thia-1-azabicyclo[5.2.0]nonan-9-on in 500 ml Methylenchlorid hinzugefügt.

Die Mischung wird dann 1 Stunde bei 0° gerührt und mit gesättigter wässriger Natriumbicarbonatlösung, 10%-iger Natriumbisulfitlösung und wiederum mit gesättigter wässriger Natriumbicarbonatlösung gewaschen. Die organische Schicht wird über Natriumsulfat getrocknet und filtriert. Das Lösungsmittel wird durch Verdampfung entfernt. Der erhaltene Rückstand ist ein Feststoff, der aus Isopropanol umkristallisiert wird. Schmelzpunkt 158°.

Beispiel 6: (5R,7R,8S)-2,2,5-Trimethyl-8-(4-nitrobenzyloxycarbonyloxymethyl)-3-oxa-6-thia-1-azabicyclo[5.2.0]nonan-9-on-6-dioxid

Diese Verbindung wird in analoger Weise zu Beispiel 5 durch Umsetzen von (5R,7R,8S)-2,2,5-Trimethyl-8-(4-nitrobenzyloxycarbonyloxymethyl)-3-oxa-6-thia-1-azabicyclo[5.2.0]nonan-9-on mit m-Chlorperbenzoesäure erhalten.

Beispiel 7: trans-3-(4-Nitrobenzyloxycarbonyloxymethyl)-4-(2-hydroxyäthylsulfonyl)-azetidin-2-on

4,35 g (10 mMol) trans-2,2-Dimethyl-8-(4-nitrobenzyloxycarbonyloxymethyl)-3-oxa-6-thia-1-azabicyclo[5.2.0] nonan-9-on-6-dioxid werden in 130 ml Eisessig gelöst und mit 30 ml Wasser verdünnt. Die Temperatur der Mischung wird während eines Zeitraums von 4 Tagen auf 55° gehalten. Das Lösungsmittel wird durch Verdampfung entfernt. Der Rückstand wird in Aethylacetat gelöst und mit 30 ml wässriger Natriumbicarbonatlösung gewaschen. Die organische Schicht wird abgetrennt. Die wässrige Schicht wird mit Aethylacetat extrahiert. Die organische Phase wird über Natriumsulfat getrocknet und filtriert. Das Lösungsmittel wird durch Verdampfen entfernt. Es wird ein viskoser Rückstand erhalten. Die Reinigung durch Chromatographie und Umkristallisation aus Isopropanol ergibt einen Feststoff. Schmelzpunkt 128°.

Beispiel 8: (3S,4R)-3-(4-Nitrobenzyloxycarbonyloxymethyl)-4-[(2R)-1-hydroxy-2-propylsulfonyl]-azetidin-2-on

Diese Verbindung wird in analoger Weise zum Beispiel 7 durch Hydrolysieren von (5R,7R,8S)-2,2,5-Trimethyl-8-(4-nitrobenzyloxycarbonyloxymethyl)-3-oxa-6-thia-1-azabicyclo[5.2.0]nonan-9-on-6-dioxid mit einer Lösung von Eisessig in Wasser erhalten.

Beispiel 9: trans-3-(4-Nitrobenzyloxycarbonyloxymethyl)-4-[5-(4-nitrobenzyloxycarbonylamino)-valeroylthio]-azetidin-2-on

Zu einer während 30 Minuten gerührten Suspension von trans-3-(4-Nitrobenzyloxycarbonyloxymethyl)-4-(2-hydroxyäthylsulfonyl)-azetidin-2-on (8,536 g, 22 mMol) in 14,5 ml Acetonitril, 29 ml Aceton und 145,2 ml Phosphatpuffer pH 8 wird unter Rühren innerhalb 60 Minuten bei Zimmertemperatur ein Gemisch von 5-(4-Nitrobenzyloxycarbonylamino)-thiovaleriansäure, 26,4 ml 1N NaOH, 26,4 ml Wasser und 6 ml Acetonitril zugetropft. Das Gemisch wird während genau 90 Minuten bei Zimmertemperatur nachgerührt und die entstandene milchige Emulsion in 250 ml Essigester aufgenommen. Die wässrige Phase wird noch mit zwei Portionen von je 250 ml Essigester extrahiert, die Extrakte vereinigt und über $Na_2SO_4$ getrocknet. Filtration und Eindampfen des Lösungsmittels im Vakuum liefert ein gelbliches viskoses Oel. Chromatographie auf 240 g Merck-Kieselgel mit $CH_2Cl_2$-EtOAc (3:1) liefert reines amorphes Produkt. IR in $CH_2Cl_2$: 3650, 3000, 1790, 1760, 1730, 1520, 1340, 1220 $cm^{-1}$.

Das Ausgangsmaterial kann wie folgt hergestellt werden:

a) 5-(4-Nitrobenzyloxycarbonylamino)-valeriansäure

Zu einer Lösung von 5-Aminovaleriansäure (11,7 g, 0,1 Mol) in 100 ml 1N NaOH-Lösung wird bei Raumtemperatur fester Chlorameisensäure-4-nitrobenzylester (28,0 g, 0,13 Mol) gegeben und die entstandene

0082113

beige Suspension innert 40 Minuten mit 120 ml 1N NaOH-Lösung versetzt. Anschliessend wird das Reaktionsgemisch während 38 Stunden bei Raumtemperatur gerührt. Das Gemisch wird mit zwei Portionen von 180 ml $CHCl_3$ gewaschen und die wässrige Phase mit 5N HCl auf pH 2 gestellt. Die weisse Suspension wird filtriert und der Rückstand im Hochvakuum getrocknet. Das Filtrat wird mit $CHCl_3$ (2 x 250 ml) extrahiert, die organische Phase über $Na_2SO_4$ getrocknet und im Vakuum eingedampft. Der Rückstand wird mit dem Filterrückstand vereinigt. Kristallisation aus 200 ml heissem Isopropanol liefert nach 3 Tagen bei 0° reines kristallines Produkt, Smp. 85-87°.

b) <u>5-(4-Nitrobenzyloxycarbonylamino)-thiovaleriansäure</u>

5-(4-Nitrobenzyloxycarbonylamino)-valeriansäure (59,25 g, 0,2 Mol) wird in Methylenchlorid (300 ml) suspendiert und durch Zugabe von Triäthylamin (61,2 ml, 0,44 Mol) gelöst. Zu dieser Lösung wird unter Rühren bei -10° innert 30 Minuten eine Lösung von Chlorameisensäureisobutylester (28,8 ml) in Methylenchlorid (60 ml) zugetropft. Nach Zugabe wird noch während 30 Minuten bei 0° nachgerührt. Dann wird während 60 Minuten $H_2S$ eingeleitet bei 0°. Nach Entfernen des überschüssigen $H_2S$ mit Stickstoff wird die Suspension mit $CHCl_3$ (1,5 l) gelöst, mit zwei Portionen von je 250 ml wässriger 2N HCl gewaschen. Die organische Phase wird mit 600 ml gesättigter Natriumbicarbonatlösung extrahiert und die wässrige Phase im Erlenmeyer unter Rühren vorsichtig mit 6N wässriger HCl sauer gestellt. Die wässrige Lösung wird mit zwei Portionen von je 1 l $CHCl_3$ extrahiert und die organische Phase über $Na_2SO_4$ getrocknet und dann filtriert. Eindampfen des Lösungsmittels im Vakuum liefert das Produkt als gelbes Oel,das im Kühlschrank kristallisiert, Smp. ca. 35°.

<u>Beispiel 10</u>: <u>(3S,4R)-3-(4-Nitrobenzyloxycarbonyloxymethyl)-4-[5-(4-nitrobenzyloxycarbonylamino)-valeroylthio]-azetidin-2-on</u>

Nach der gleich Vorschrift, wie in Beispiel 9 beschrieben, wird ausgehend von (3S,4R)-3-(4-Nitrobenzyloxycarbonyloxymethyl)-4-[(2R)-1-hydroxy-2-propylsulfonyl]-azetidin-2-on die Titelverbindung mit identi-

schem IR-Spektrum erhalten. $[\alpha]_D^{20}$ + 61° (c=1, $CHCl_3$).

Beispiel 11: 2-[trans-3-(4-Nitrobenzyloxycarbonyloxymethyl)-4-(5-(4-nitrobenzyloxycarbonylamino)-valeroylthio)-2-oxo-azetidinyl]-2-hydroxyessigsäure-4-nitrobenzylester

Ein Gemisch von 4,79 g (8,11 mMol) trans-3-(4-Nitrobenzyloxycarbonyloxymethyl)-4-[5-(4-nitrobenzyloxycarbonylamino)-valeroylthio]-azetidin-2-on und 3,10 g (12,16 mMol) frisch zubereitetes Glyoxylsäure-4-nitrobenzylester-äthylhemiketal in 32 ml Toluol und 8 ml N,N-Dimethylformamid wird mit 16,3 g Molekularsieb 4 Å versetzt und bei 40° während 20 Stunden gerührt. Die Mischung wird filtriert und der Filterrückstand mit Essigester gewaschen. Eindampfen des Filtrats und Trocknung bei 40° im Hochvakuum ergibt ein gelbliches viskoses Oel (8,19 g). Chromatographie auf 350 g Merck-Kieselgel mit Toluol-Essigester (7:1) und Toluol-Essigester (2:1) ergibt die reine Titelverbindung als amorphe Festkörper. IR in $CH_2Cl_2$: 3440, 2940, 1780, 1750, 1725, 1515, 1350, 1230 $cm^{-1}$.

Beispiel 12: 2-[(3S,4R)-3-(4-Nitrobenzyloxycarbonyloxymethyl)-4-(5-(4-nitrobenzyloxycarbonylamino)-valeroylthio)-2-oxo-azetidinyl]-2-hydroxyessigsäure-4-nitrobenzylester

Nach der gleichen Vorschrift, wie in Beispiel 11 beschrieben, wird ausgehend von (3S,4R)-3-(4-Nitrobenzyloxycarbonyloxymethyl)-4-[5-(4-nitrobenzyloxycarbonylamino)-valeroylthio]-azetidin-2-on die Titelverbindung mit identischem IR-Spektrum erhalten.

Beispiel 13: 2-[trans-3-(4-Nitrobenzyloxycarbonyloxymethyl)-4-(5-(4-nitrobenzyloxycarbonylamino)-valeroylthio)-2-oxo-azetidinyl]-2-chloressigsäure-4-nitrobenzylester

Zu einer Lösung von 9,6 g (12,5 mMol) 2-[trans-3-(4-Nitrobenzyloxycarbonyloxymethyl)-4-(5-(4-nitrobenzyloxycarbonylamino)-valeroylthio)-2-oxo-azetidinyl]-2-hydroxyessigsäure-4-nitrobenzylester in 64 ml Tetrahydrofuran werden unter Rühren bei -15° nacheinander 1,76 ml (24,2 mMol) Thionylchlorid und eine Lösung von 3,4 ml Triäthylamin in 5,6 ml Tetrahydrofuran je innert 15 Minuten zugetropft. Die weisse Suspension wird noch bei 0° während 30 Minuten gerührt, mit 360 ml Methylenchlorid versetzt, mit 60 ml 0,1 N wässriger HCl-Lösung und dreimal mit je 100 ml gesättigter NaCl-Lösung gewaschen. Die Lösung wird über $Na_2SO_4$ getrocknet und filtriert. Eindampfen des Lösungsmittels im Vakuum ergibt das reines Produkt als leicht gelblichen Festkörper. IR in $CH_2Cl_2$: 3440, 2940, 1785, 1750, 1720, 1520, 1345, 1230 $cm^{-1}$.

Beispiel 14: 2-[(3S,4R)-3-(4-Nitrobenzyloxycarbonyloxymethyl)-4-(5-(4-nitrobenzyloxycarbonylamino)-valeroylthio)-2-oxo-azetidinyl]-2-chloressigsäure-4-nitrobenzylester

Nach der gleichen Vorschrift wird wie in Beispiel 13 beschrieben, ausgehend von 2-[(3S,4R)-3-(4-Nitrobenzyloxycarbonyloxymethyl)-4-(5-(4-nitrobenzyloxycarbonylamino)-valeroylthio)-2-oxo-azetidinyl]-2-hydroxyessigsäure-4-nitrobenzylester die Titelverbindung mit identischem IR-Spektrum erhalten.

Beispiel 15: 2-[trans-3-(4-Nitrobenzyloxycarbonyloxymethyl)-4-(5-(4-nitrobenzyloxycarbonylamino)-valeroylthio)-2-oxo-azetidinyl]-2-triphenylphosphoranylidenessigsäure-4-nitrobenzylester

Eine Lösung von 3,7 g (4,67 mMol) 2-[3-(4-Nitrobenzyloxycarbonyloxymethyl)-4-(5-(4-nitrobenzyloxycarbonylamino)-valeroylthio)-2-oxo-

azetidinyl]-2-chloressigsäure-4-nitrobenzylester und 2,79 g Triphenylphosphin (10,65 mMol) in 3,9 ml Tetrahydrofuran wird unter Stickstoff während zwei Tagen bei 5° stehengelassen. Man verdünnt mit 200 ml Methylenchlorid, wäscht mit 50 ml gesättigter wässriger $NaHCO_3$-Lösung, trocknet die organische Phase über $Na_2SO_4$ und filtriert. Eindampfen des Lösungsmittels im Vakuum ergibt 6,6 g rötliches Oel. Chromatographie auf 170 g Merck-Kieselgel mit Toluol-Essigester (2:1) ergibt ein gelbliches Oel. IR in $CH_2Cl_2$: 2940, 2440, 1755, 1725, 1515, 1355, 1230 $cm^{-1}$.

Beispiel 16: 2-[(3S,4R)-3-(4-Nitrobenzyloxycarbonyloxymethyl)-4-(5-(4-nitrobenzyloxycarbonylamino)-valeroylthio)-2-oxo-azetidinyl]-2-triphenylphosphoranylidenessigsäure-4-nitrobenzylester

Nach der gleichen Vorschrift wird, wie in Beispiel 15 beschrieben, ausgehend von 2-[(3S,4R)-3-(4-Nitrobenzyloxycarbonyloxymethyl)-4-(5-(4-nitrobenzyloxycarbonylamino)-valeroylthio)-2-oxo-azetidinyl]-2-chloressigsäure-4-nitrobenzylester die Titelverbindung mit identischem IR-Spektrum erhalten. $[\alpha]_D^{20}$ + 14° (c=1, $CHCl_3$)

Beispiel 17: trans-6-(4-Nitrobenzyloxycarbonyloxymethyl)-2-[4-(4-nitrobenzyloxycarbonylamino)-butyl]-penem-3-carbonsäure-4-nitrobenzylester

Eine Lösung von 8,5 g (8,4 mMol) 2-[trans-3-(4-Nitrobenzyloxycarbonyloxymethyl)-4-(5-(4-nitrobenzyloxycarbonylamino)-valeroylthio)-2-oxo-azetidinyl]-2-triphenylphosphoranylidenessigsäure-4-nitrobenzylester in 2,6 l Toluol wird unter Stickstoffatmosphäre während 25 Std. am Rückfluss gekocht. Eindampfen des Lösungsmittels und Chromatographie des Rückstandes an 260 g Kieselgel mit Toluol-Essigester (3:1) ergibt das reine Produkt als farblosen amorphen Festkörper. IR in $CH_2Cl_2$: 3040, 2940, 1790, 1750, 1720, 1605, 1520, 1345, 1230 $cm^{-1}$.

Beispiel 18: (5R,6S)-6-(4-Nitrobenzyloxycarbonyloxymethyl)-2-[4-(4-nitrobenzyloxycarbonylamino)-butyl]-penem-3-carbonsäure-4-nitrobenzylester

Nach der gleichen Vorschrift, wie in Beispiel 17 beschrieben wird ausgehend von 2-[(3S,4R)-3-(4-Nitrobenzyloxycarbonyloxymethyl)-4-(5-(4-nitrobenzyloxycarbonylamino)-valeroylthio)-2-oxo-azetidinyl]-2-triphenylphosphoranylidenessigsäure-4-nitrobenzylester die Titelverbindung mit identischem IR Spektrum erhalten. $[\alpha]_D^{20}$ + 36° (c= 1, $CHCl_3$)

Beispiel 19: trans-6-Hydroxymethyl-2-(4-aminobutyl)-penem-3-carbonsäure

Ein Gemisch von 190 mg (0,25 mMol) trans-6-(4-Nitrobenzyloxycarbonyloxymethyl)-2-[4-(4-nitrobenzyloxycarbonylamino)-butyl]-penem-3-carbonsäure-4-nitrobenzylester, 24 ml Dioxan und 5 ml 0,1 N wässriger HCl-Lösung wird in einer Hydrierente - mit parallelgeschalteter Flasche mit KOH zur $CO_2$-Absorption - bei Zimmertemperatur und Normaldruck über 200 mg 10 % Pd auf Kohle während 90 Min. hydriert. Während dieser Zeit wird 39 ml $H_2$ aufgenommen. Das Gemisch wird durch Cellite filtriert, im Vakuum auf ein Volumen von 5 ml eingedampft und die entstandene gelbe Suspension mit einer Lösung von 42 mg $NaHCO_3$ in 1 ml Wasser versetzt. Die Lösung wird auf sechs "reverse phase" Dünnschichtchromatographie-Platten (L 254 OPTI-UP C-12-20, Hersteller ANTEC AG, Bennwil, Schweiz) aufgetragen. Chromatographie mit $CH_3CN$ - $H_2O$ 2:3 und Eluieren der mobilen UV-aktiven Fraktion mit $CH_3CN$ - $H_2O$ (4:1), teilweises Eindampfen des Eluats und Lyophilisation der zurückbleibenden wässrigen Lösung liefert einen farblosen amorphen Festkörper.
UV in $H_2O$ (pH 3): $\lambda_{max}$ 256 nm (2500), 318 nm (5600).

Beispiel 20: (3S,5R,6R)-2,2-Dimethyl-6-(tert.-butyl-dimethylsilyloxymethyl)-penam-3-carbonsäuremethylester-1,1-dioxid

Eine Lösung von 23,6 g (85 mMol) (3S,5R,6R)-2,2-Dimethyl-6-hydroxymethyl-penam-3-carbonsäuremethylester-1,1-dioxid in 50 ml Dimethylformamid wird mit 25,5 g (170 mMol) tert.-Butyl-dimethylchlorsilan und 11,5 g (170 mMol) Imidazol bei Raumtemperatur während 45 Minuten gerührt. Dann wird das Lösungsmittel am Hochvakuum abdestilliert und der Rückstand in Aethylacetat aufgenommen. Die Lösung wird mit 1N-Schwefelsäure und dann mit Wasser gewaschen und die wässerigen Lösungen zweimal mit Aethylacetat extrahiert. Die organische Phase wird mit Natriumsulfat getrocknet und am Rotationsverdampfer eingeengt. Das Produkt fällt als kristalline Masse an.

DC : Silicagel, Toluol/Aethylacetat (4:1): Rf = 0,56

IR : ($CH_2Cl_2$) 3,4; 5,57; 5,65 µm.

Beispiel 21: 2-[(3S,4R)-3-(tert.-Butyl-dimethylsilyloxymethyl)-4-methylsulfonyl-2-oxo-azetidin-1-yl]-3-methyl-2-butensäuremethylester

Eine Lösung von 202 g (0,51 Mol) (3S,5R,6R)-2,2-Dimethyl-6-(tert.-butyldimethylsilyloxymethyl)-penam-3-carbonsäuremethylester-1,1-dioxid in 800 ml Tetrahydrofuran werden mit 9 ml DBU versetzt und während 5 Minuten bei Raumtemperatur gerührt. Dann wurden weitere 95 ml DBU zugegeben und 30 Minuten bei Raumtemperatur gerührt. Anschliessend fügte man unter Kühlung 42,3 ml (0,68 Mol) Methyljodid zu. Nach 3 Stunden Reaktionsdauer wird vom auskristallisierten DBU-Hydrojodid abfiltriert und das Filtrat eingeengt. Der Rückstand wird in Aethylacetat aufgenommen und die Lösung mit 1N-Schwefelsäure, Wasser und Natriumbicarbonatlösung gewaschen. Die wässerigen Phasen werden zweimal mit Aethylacetat extrahiert. Die vereinigten organischen Phasen werden über Natriumsulfat getrocknet und die Lösung zu einem dicken Oel eingeengt.

DC : Silicagel, Toluol/Aethylacetat (4:1); Rf = 0,42

IR : ($CH_2Cl_2$) 5,63; 5,81; 6,17 µm.

Beispiel 22: (3S,4R)-3-Hydroxymethyl-4-methylsulfonyl-azetidin-2-on und (3S,4R)-3-(tert.Butyl-dimethylsilyloxymethyl)-4-methylsulfonyl-azetidin-2-on

Eine Lösung von 25 g (61,7 mMol) 2-[(3S,4R)-3-(tert.-Butyl-dimethylsilyloxymethyl)-4-methylsulfonyl-2-oxo-azetidin-1-yl]-3-methyl-2-butensäuremethylester in 400 ml Methylenchlorid wird bei -10° mit einem Ozon/

BAD ORIGINAL

Sauerstoffgemisch behandelt. Das Verschwinden des Ausgangsmaterials wird dünnschichtchromatographisch kontrolliert. Nach Beendigung der Reakion werden 30 ml Dimethylsulfid zugegeben und für 3 Stunden bei Raumtemperatur weitergerührt. Die Lösung wird eingeengt und der Rückstand in einem Gemisch von 160 ml Methanol, 24 ml Aethylacetat und 3 ml Wasser aufgenommen und während 40 Minuten auf 70° erwärmt. Das Lösungsmittel wird anschliessend abgezogen und der Rückstand zweimal mit Toluol abgezogen. Das kristallisierende Oel wird in Methylenchlorid aufgenommen und die Kristalle bestehend aus (3S),(4R)-3-Hydroxymethyl-4-methylsulfonyl-azetidin-2-on, durch Filtration isoliert. Das Filtrat wird eingeengt und (3S,4R)-3-(tert.Butyl-dimethylsilyloxymethyl)-4-methylsulfonyl-azetidin-2-on durch Chromatographie an Silicagel mit Toluol/Aethylacetat (3:1) in reiner Form erhalten:

(3S,4R)-3-Hydroxymethyl-4-methylsulfonyl-azetidin-2-on: DC, Silicagel, Toluol/Aethylacetat (1:1); Rf = 0,36, IR: ($CH_2Cl_2$) 2,96; 3,54; 5,61 µm.

(3S,4R)-3-(tert.Butyl-dimethylsilyloxymethyl)-4-methylsulfonyl-azetidin-2-on: DC; Silicagel, Toluol/Aethylacetat (1:1) Rf = 0,06.

Eine Lösung von 14,6 g (81,5 mMol) (3S,4R)-3-Hydroxymethyl-4-methylsulfonyl-azetidin-2-on in 40 ml Dimethylformamid wird mit 24 g (183 mMol) tert.-Butyldimethylchlorsilan und 11 g (163 mMol) Imidazol während 45 Minuten bei Raumtemperatur versetzt. Dann wird das Lösungsmittel am Hochvakuum abgezogen und der Rückstand in Aethylacetat aufgenommen. Die organische Phase wird nacheinander mit 1N-Schwefelsäure, Wasser und Natriumbicarbonatlösung gewaschen. Die wässerigen Phasen werden zweimal mit Aethylacetat extrahiert. Die vereinigten organischen Phasen werden über Natriumsulfat getrocknet und am Rotationsverdampfer eingeengt. Der kristalline Rückstand ist reines (3S,4R)-3-(tert.-Butyl-dimethylsilyloxymethyl)-4-methylsulfonyl-azetidin-2-on.

Beispiel 23: (3S,4R)-3-(tert.Butyl-dimethylsilyloxymethyl)-4-(5-allyloxycarbonylaminovaleroylthio)-azetidin-2-on

Eine Aufschlämmung von 7,0 g (32,2 mMol) 5-Allyloxycarbonylamino-thiovaleriansäure in 50 ml Wasser wird auf 0° gekühlt und mit 1N Natronlauge bis pH 8 versetzt. Anschliessend werden 30 ml Methylenchlorid, 2,93 g (10 mMol) 3-(tert.Butyldimethylsilyloxymethyl)-4-methylsulfonylazetidin-2-on und 278 mg (1 mMol) Tetrabutylammoniumchlorid zugefügt und das zweiphasige System bei 0° während 3 Stunden gut gerührt. Die Phasen werden getrennt und je einmal mit gesättigter $NaHCO_3$-Lösung und gesättigter NaCl-Lösung gewaschen. Die wässrigen Phasen werden noch zweimal mit Methylenchlorid extrahiert, die Extrakte vereinigt und über $Na_2SO_4$ getrocknet. Eindampfen des Lösungsmittels im Vakuum lieferte ein Rohprodukt, welches an 50 g Silicagel mit Toluol und Aethylacetat 2:1 als Laufmittel chromatographiert wird.
DC (Silicagel) Toluol/Aethylacetet (1:1); Rf = 0,51
IR (Methylenchlorid): 2,90; 2,94; 5,68; 5,88 $\mu$.

Das Ausgangsmaterial kann wie folgt hergestellt werden:

6,75 g (33,6 mMol) 5-Allyloxycarbonylamino-valeriansäure wurde in 40 ml Methylenchlorid gelöst und mit 10,2 ml (73,8 mMol) Triäthylamino versetzt. Zu dieser Lösung wird unter Rühren bei -10° innert 30 Minuten eine Lösung von 5,2 ml (40,3 mMol) Chlorameisensäure-isobutylester in 7 ml Methylenchlorid zugetropft. Nach Zugabe wird noch 30 Minuten bei -10° nachgerührt. Dann wird während 60 Minuten bei 0° $H_2S$ eingeleitet. Die Lösung wird mit 1N Schwefelsäure gewaschen, anschliessend erst mit 50 ml und dann mit 30 ml gesättigter Natriumbicarbonatlösung extrahiert und die wässrige Phase im Erlenmeyer-Kolben unter Rühren vorsichtig mit 20%iger Phosphorsäure sauer gestellt. Die wässrige Lösung wird dreimal mit Chloroform extrahiert und die organische Phase über $Na_2SO_4$ getrocknet und filtriert. Eindampfen des Lösungsmittels im Vakuum und Trocknen im Hochvakuum liefert 5-Allyloxy-

BAD ORIGINAL

carbonylamino-thiovaleriansäure als Oel.
IR (Methylenchlorid): 2,94; 3,95; 5,97; 6.75 µ

Beispiel 24: 2-[(3S,4R)-3-(tert.Butyl-dimethylsilyloxymethyl)-4-(5-allyloxycarbonylaminovaleroylthio)-2-oxo-azetidin-1-yl]-2-hydroxyessigsäureallylester

Ein Gemisch von 1,9 g (4,4 mMol) (3S,4R)-3-(tert.Butyldimethylsilyloxymethyl)-4-(5-allyloxycarbonylaminovaleroylthio)-azetidin-2-on und 1,9 g (13,2 mMol) Glyoxylsäure-allylester-äthylhemiketal in 30 ml Toluol und 2 ml N,N-Dimethylformamid wird mit 16 g Molekularsieb 4Å versetzt und bei Raumtemperatur während 16 Stunden gerührt. Das Gemisch wird filtriert und der Filterrückstand mit Toluol gewaschen. Eindampfen des Filtrats und Trocknung bei 40° im Hochvakuum ergibt das Produkt als gelbes Oel.
DC (Silicagel) Toluol/Aethylacetat (2:1); Rf = 0,44;
IR (Methylenchlorid): 2,85, 2,91, 5,65, 5,74, 5,81 µ.

Beispiel 25: 2-[(3S,4R)-3-(tert.Butyl-dimethylsilyloxymethyl)-4-(5-allyloxy-carbonylaminovaleroylthio)-2-oxo-azetidin-1-yl]-2-triphenylphosphoranylidenessigsäureallylester

Zu einer Lösung von 2,35 g (4,32 mMol) 2-[(3S,4R)-3-(tert.Butyl-dimethylsilyloxymethyl)-4-(5-allyloxycarbonylaminovaleroylthio)-2-oxo-azetidin-1-yl]-2-hydroxyessigsäureallylester in 60 ml Tetrahydrofuran werden unter Rühren bei -20° nacheinander 0,37 ml (5,14 mMol) Thionylchlorid und 0,7 ml (5,14 mMol) Triäthylamin innert 5 Minuten zugegeben. Die weisse Suspension wird noch bei -15° während 20 Minuten gerührt, mit Methylenchlorid versetzt, mit 0,1 N Salzsäure und zweimal mit gesättigter NaCl-Lösung gewaschen. Die Lösung wird über $Na_2SO_4$ getrocknet, filtriert und im Vakuum eingeengt. Der Rückstand wird in 7 ml Tetrahydrofuran gelöst, mit 1,8 g (6,8 mMol) Triphenylphosphin und 0,6 ml (4,4 mMol) Triäthylamin versetzt und bei Raumtemperatur während 16 Stunden gerührt. Man verdünnt mit Methylenchlorid, wäscht

mit gesättigter wässriger $NaHCO_3$-Lösung, trocknet die organische Phase über $Na_2SO_4$ und filtriert. Eindampfen des Lösungsmittels im Vakuum und Chromatographie des Rückstandes an 50 g Silicagel mit Toluol/Aethylacetat 2:1 ergibt das reine Produkt.

DC (Silicagel) Toluol/Aethylacetat (1:1), Rf = 0,40

IR (Methylenchlorid): 2,91; 5,71; 5,83; 5,95; 6,21 μ.

<u>Beispiel 26:</u> <u>2-[(3S,4R)-4-(5-Allyloxycarbonylaminovaleroylthio)-3-hydroxymethyl-2-oxo-azetidin-1-yl]-2-triphenylphosphoranyliden-essigsäureallylester</u>

Eine Lösung von 546 g (0,69 mMol) 2-[(3S,4R)-3-(tert.Butyl-dimethylsilyloxymethyl)-4-(5-allyloxycarbonylaminovaleroylthio)-2-oxo-azetidin-1-yl]-2-triphenylphosphoranyliden-essigsäureallylester in 12 ml Tetrahydrofuran wird auf -10° gekühlt, mit 536 mg (1,7 mMol) Tetrabutylammoniumfluorid versetzt und 15 Minuten gerührt. Dann wird mit Methylenchlorid verdünnt und mit wässriger $NaHCO_3$-Lösung und NaCl-Lösung gewaschen. Die organische Phase wird über $Na_2SO_4$ getrocknet und im Vakuum eingeengt. Der Rückstand wird auf Silicagel mit dem Laufmittel Aethylacetat chromatographiert

DC (Silicagel) Aethylacetat: Rf = 0,24

IR (Methylenchlorid): 2,79; 2,90; 5,71; 5,83; 6,21 μ.

<u>Beispiel 27:</u> <u>(5R,6S)-2-(4-Allyloxycarbonylaminobutyl)-6-hydroxymethyl-2-penem-3-carbonsäureallylester</u>

Eine Lösung von 98 mg (0,14 mMol) 2-[(3S,4R)-4-(5-Allyloxycarbonylaminovaleroylthio)-3-hydroxymethyl-2-oxo-azetidin-1-yl]-2-triphenylphosphoranyliden-essigsäureallylester in 10 ml Toluol wird unter Stickstoffatmosphäre während 16 Stunden auf 105° erwärmt. Eindampfen des Lösungsmittels und Chromatographie des Rückstandes auf einer präparativen Silicagelplatte mit Toluol/Aethylacetat (1:1) ergibt das reine Produkt.

DC (Silicagel) Aethylacetat; Rf = 0,55;

IR (Methylenchlorid): 2,76; 5,58; 5,81; 6,06 μ.

Beispiel 28: (5R,6S)-2-(4-Aminobutyl)-6-hydroxymethyl-2-penem-3-carbonsäure

Nach der gleichen Vorschrift, wie in Beispiel 19 beschrieben, wird ausgehend von (5R,6S)-6-(4-Nitrobenzyloxycarbonyloxymethyl)-2-[4-(4-nitrobenzyloxycarbonylamino)-butyl]-2-penem-3-carbonsäure-4-nitrobenzylester das Produkt erhalten. $[\alpha]_D^{20}$ + 58° (c=1, $H_2O$/NaOH). UV in $H_2O$ (pH 3): $\lambda_{max}$ 256 nm (2500), 318 nm (5600).

Das gleiche Produkt kann auch wie folgt hergestellt werden:

Eine Lösung von 46 mg (0,11 mMol) (5R,6S)-2-(4-Allyloxycarbonylaminobutyl)-6-hydroxymethyl-2-penem-3-carbonsäureallylester in 2 ml Methylenchlorid und 1 ml Diäthyläther wird bei Raumtemperatur mit 20 mg (0,14 mMol) 2-Aethylhexansäure, 10 mg Triphenylphosphin und 6 mg Tetrakis-(triphenylphosphin)-palladium während 16 Stunden gerührt. Die Lösung wird mit Methylenchlorid verdünnt und mit Wasser zweimal gewaschen. Die vereinigten wässrigen Phasen werden lyophilisiert und der Rückstand auf "reversed phase" Dünnschichtplatten (Opti-$UPC_{12}$) chromatographiert (Acetonitril/Wasser 2:3). Eluieren der entsprechenden Zone mit Acetonitril/Wasser (4:1), teilweises Eindampfen des Eluats und Lyophilisation der zurückbleibenden wässrigen Lösung liefert ebenfalls das Endprodukt mit identischen physikalischen Daten.

Beispiel 29: Natriumsalz der (5R,6S)-2-[4-(1-Aethoxycarbonylprop-1-en-2-ylamino)-butyl]-6-hydroxymethyl-2-penem-3-carbonsäure

0,139 ml (1,1 mMol) Acetessigsäureäthylester werden zu einer Suspension von 0.27 mg (1 mMol) (5R,6S)-2-(4-Aminobutyl)-6-hydroxymethyl-2-penem-3-carbonsäure und 0,5 ml 2M-Natronlauge in 3 ml Isopropanol gegeben und 3 Stunden bei Raumtemperatur gerührt. Durch Zugabe von Diäthyläther wird das Produkt ausgefällt. IR-Spektrum (Nujol): Absorptionsbanden bei 3,0; 5,58; 5,75 $\mu$.

Beispiel 30: (5R,6S)-2-(4-Aminobutyl)-6-hydroxymethyl-2-penem-3-carbonsäure-1-äthoxycarbonyloxyäthylester

1,2 g Natriumjodid werden in 3,7 ml Aceton gelöst und mit 0,275 ml Aethyl-1-chloräthylcarbonat versetzt. Das Gemisch wird bei Raumtemperatur während 3 Stunden gerührt. Anschliessend wird die Lösung auf 15,0 ml Methylenchlorid getropft und von den ausgefallenen anorganischen Salzen abfiltriert. Die Methylenchloridlösung wird bis auf 2 ml eingeengt und bei 0° zu einer Lösung von 0,27 g (1 mMol) (5R,6S)-2-(4-Aminobutyl)-6-hydroxymethyl-2-penem-3-carbonsäure in 4 ml Dimethylacetamid gegeben. Dann wird während 3 Stunden bei 0° gerührt, anschliessend mit Aethylacetat verdünnt und dreimal mit Wasser gewaschen. Die organischen Phasen werden über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): Absorptionsbanden bei 5,58; 5,75 $\mu$.

Beispiel 31: (5R,6S)-2-(4-Aminobutyl)-6-hydroxymethyl-2-penem-3-carbonsäure-pivaloyloxymethylester

0,6 g Natriumjodid werden in 2 ml Aceton gelöst und mit 0,15 ml Pivalinsäurechlormethylester versetzt. Das Gemisch wird bei Raumtemperatur während 3 Stunden gerührt und anschliessend auf 7,5 ml Methylenchlorid getropft. Die ausgefallenen anorganischen Salze werden abfiltriert. Die Methylenchloridlösung wird bis auf 1 ml eingeengt und zu einer Lösung von 0,1 g (0,4 mMol)(5R,6S)-2-(4-Aminobutyl)-6-hydroxymethyl-2-penem-3-carbonsäure und 0,07 ml Diisopropyläthylamin in 4 ml N,N-Dimethylacetamid bei 0° gegeben. Dann wird während 3 Stunden bei 0° gerührt, anschliessend mit Aethylacetat verdünnt und dreimal mit Wasser gewaschen. Die organische Phase wird über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): Absorptionsbanden bei 5,58; 5,75 $\mu$.

Beispiel 32: Trockenampullen oder Vials, enthaltend 0,5 g (5R,6S)-2-(4-Aminobutyl)-6-hydroxymethyl-2-penem-3-carbonsäure als Wirksubstanz werden wie folgt hergestellt:

Zusammensetzung (für 1 Ampulle oder Vial):

| | |
|---|---|
| Wirksubstanz | 0,5 g |
| Mannit | 0,05 g. |

Eine sterile wässrige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Patentansprüche (für alle benannten Länder ausser Oesterreich)

1. 2-Aminobutyl-6-hydroxymethyl-2-penem-Verbindungen der Formel

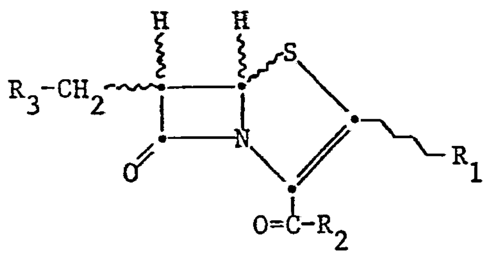

(I)

worin $R_1$ eine gegebenenfalls substituierte Aminogruppe darstellt, $R_2$ Hydroxy oder einen zusammen mit der Carbonylgruppe -C(=O)- eine geschützte Carboxylgruppe bildenden Rest $R_2^A$ bedeutet und $R_3$ eine gegebenenfalls geschützte Hydroxygruppe ist, ihre Salze, ihre optischen Isomeren und Mischungen ihrer optischen Isomeren.

2. Verbindungen der Formel I gemäss Patentanspruch I, worin $R_1$ Amino, 1-Acyl-niederalk-1-en-2-yl-amino oder eine mono- oder disubstituierte Methylenaminogruppe bedeutet, $R_2$ Hydroxy oder zusammen mit der Carbonylgruppe eine unter physiologischen Bedingungen spaltbare veresterte Carboxylgruppe darstellt und $R_3$ Hydroxy ist, ihre Salze, ihre optischen Isomeren und Mischungen ihrer optischen Isomeren.

3. Verbindungen der Formel I gemäss Patentanspruch 1, worin $R_1$ Amino ist, $R_2$ Hydroxy, Niederalkanoyloxymethoxy, Aminoniederalkanoyloxymethoxy, Phthalidyloxy, 4-Crotonolactonyloxy, 4-Butyrolacton-4-yloxy, Indanyloxy, 1-Niederalkoxy-niederalkoxy oder 1-Niederalkoxycarbonyloxy-niederalkoxy darstellt und $R_3$ Hydroxy ist, ihre pharmazeutisch verwendbaren Salze, ihre optischen Isomeren und Mischungen ihrer optischen Isomeren.

4. Verbindungen der Formel I gemäss einem der Patentansprüche 1 bis 3, worin $R_1$ Amino ist, $R_2$ Hydroxy ist und $R_3$ Hydroxy bedeutet, ihre pharmazeutisch verwendbaren Salze, ihre optischen Isomeren und Mischungen ihrer optischen Isomeren.

5. Verbindungen der Formel I gemäss Patentanspruch 1, worin deren 5-Stellung die R-Konfiguration und deren 6-Stellung die S-Konfiguration besitzt, und ihre pharmazeutisch verwendbaren Salze.

6. trans-2-(4-Aminobutyl)-6-hydroxymethyl-2-penem-3-carbonsäure und pharmazeutisch verwendbare Salze davon gemäss Patentanspruch 1.

7. (5R,6S)-2-(4-Aminobutyl)-6-hydroxymethyl-2-penem-3-carbonsäure und pharmazeutisch verwendbare Salze davon gemäss Patentanspruch 1.

8. Pharmazeutische Präparate enthaltend Verbindungen der Formel I gemäss Patentanspruch 1 oder pharmazeutisch verwendbare Salze von solchen Verbindungen.

9. Verwendung von Verbindungen der Formel I gemäss Patentanspruch 1 und pharmazeutisch verwendbarer Salze von solchen Verbindungen zur Herstellung von Arzneimitteln.

10. Verwendung von Verbindungen der Formel I gemäss Patentanspruch 9 und pharmazeutisch verwendbarer Salze von solchen Verbindungen zur Herstellung von Antibiotika.

11. Eine Verbindung gemäss einem der Ansprüche 1 bis 7 und deren pharmazeutisch verwendbare Salze zur Anwendung in einem Verfahren zur therapeutischen Behandlung des tierischen oder menschlichen Körpers.

12. Verfahren zur Herstellung von Verbindungen der Formel I gemäss Anspruch 1, ihrer Salze, ihrer optischen Isomeren und von Mischungen ihrer optischen Isomeren, dadurch gekennzeichnet, dass man eine Ylidverbindung der Formel

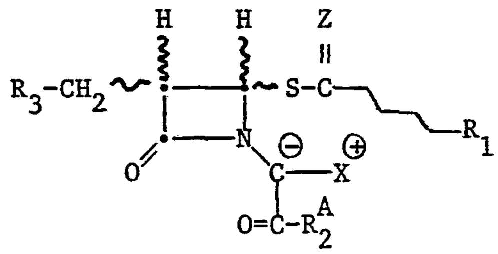

(II)

worin $R_1$ eine geschützte Aminogruppe ist, Z Sauerstoff oder Schwefel bedeutet, $X^{\oplus}$ entweder eine dreifach substituierte Phosphoniogruppe oder eine zweifach veresterte Phosphonogruppe zusammen mit einem Kation bedeutet, $R_2^A$ zusammen mit der Carbonylgruppe -C(=O)- eine geschützte Carboxylgruppe darstellt und $R_3$ eine gegebenenfalls geschützte Hydroxygruppe ist, ringschliesst, und, wenn erwünscht oder notwendig, in einer erhältlichen Verbindung der Formel I eine geschützte Aminogruppe $R_1$ in die freie oder in eine andere geschützte Aminogruppe $R_1$ überführt, und/oder, wenn erwünscht, in einer erhältlichen Verbindung der Formel I eine geschützte Carboxylgruppe $-C(=O)-R_2$ in die freie oder in eine andere geschützte Carboxylgruppe $-C(=O)-R_2$ überführt, und/oder, wenn erwünscht, in einer erhältlichen Verbindung der Formel I, worin $R_1$ Amino bedeutet, $R_1$ in eine substituierte Aminogruppe überführt, und/oder, wenn erforderlich, eine geschützte Hydroxygruppe $R_3$ in die freie Hydroxygruppe $R_3$ oder eine freie Hydroxygruppe $R_3$ in eine geschützte Hydroxygruppe $R_3$ überführt, und/oder, wenn erwünscht, eine erhältliche Verbindung mit salzbildender Gruppe in ein Salz oder ein erhaltenes Salz in die freie Verbindung oder in ein anderes Salz überführt, und/oder, wenn erwünscht, ein erhältliches Gemisch von isomeren Verbindungen in die einzelnen Isomeren auftrennt.

13. Die nach dem Verfahren gemäss Anspruch 12 erhältlichen Verbindungen.

Patentansprüche: (für Oesterreich)

1. Verfahren zur Herstellung von Verbindungen der Formel

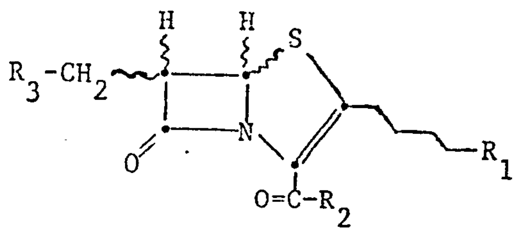

(I)

worin $R_1$ eine gegebenenfalls substituierte Aminogruppe darstellt, $R_2$ Hydroxy oder einen zusammen mit der Carbonylgruppe -C(=O)- eine geschützte Carboxylgruppe bildenden Rest $R_2^A$ bedeutet, und $R_3$ eine gegebenenfalls geschützte Hydroxygruppe ist, ihrer Salze, ihrer optischen Isomeren und von Mischungen ihrer optischen Isomeren, dadurch gekennzeichnet, dass man eine Ylid-Verbindung der Formel

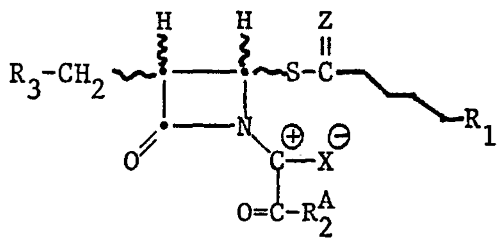

(II)

worin $R_1$ eine geschützte Aminogruppe ist, Z Sauerstoff oder Schwefel bedeutet, $X^{\oplus}$ entweder eine dreifach substituierte Phosphoniogruppe oder eine zweifach veresterte Phosphonogruppe zusammen mit einem Kation bedeutet, $R_2^A$ zusammen mit der Carbonylgruppe -C(=O)- eine geschützte Carboxylgruppe darstellt und $R_3$ eine gegebenenfalls geschütze Hydroxygruppe ist, ringschliesst, und, wenn erwünscht oder notwendig, in einer erhältlichen Verbindung der Formel I eine geschützte Aminogruppe $R_1$ in die freie oder in eine andere geschützte Aminogruppe $R_1$ überführt, und/oder, wenn erwünscht, in einer erhältlichen Verbindung der Formel I eine geschützte Carboxylgruppe $-C(=O)-R_2$ in die freie oder in eine andere geschützte Carboxylgruppe $-C(=O)-R_2$ überführt, und/oder, wenn erwünscht, in einer erhältlichen Verbindung der Formel I, worin $R_1$ Amino bedeutet, $R_1$ in eine substituierte Aminogruppe überführt, und/oder, wenn erforderlich, eine geschützte Hydroxygruppe $R_3$ in die freie Hydroxygruppe $R_3$ oder eine freie Hydroxygruppe $R_3$ in eine geschützte Hydroxygruppe $R_3$ überführt, und/oder, wenn erwünscht, eine erhältliche Ver-

bindung mit salzbildender Gruppe in ein Salz oder ein erhaltenes Salz in die freie Verbindung oder in ein anderes Salz überführt, und/oder, wenn erwünscht, ein erhältliches Gemisch von isomeren Verbindungen in die einzelnen Isomeren auftrennt.

2. Verfahren nach Anspruch 1 zur Herstellung von Verbindungen der Formel I, worin $R_1$ Amino, 1-Acyl-niederalk-1-en-2-yl-amino oder eine mono- oder disubstituierte Methylenaminogruppe bedeutet, $R_2$ Hydroxy oder zusammen mit der Carbonylgruppe eine unter physiologischen Bedingungen spaltbare veresterte Carboxylgruppe darstellt und $R_3$ Hydroxy ist, ihrer Salze, ihrer optischen Isomeren und Mischungen ihrer optischen Isomeren.

3. Verfahren nach Anspruch 1 zur Herstellung von Verbindungen der Formel I, worin $R_1$ Amino ist, $R_2$ Hydroxy, Niederalkanoyloxymethoxy, Aminoniederalkanoyloxymethoxy, Phthalidyloxy, 4-Crotonolactonyloxy, 4-Butyrolacton-4-yloxy, Indanyloxy, 1-Niederalkoxy-niederalkoxy oder 1-Niederalkoxycarbonyloxy-niederalkoxy darstellt und $R_3$ Hydroxy ist, ihrer pharmazeutisch verwendbaren Salze, ihrer optischen Isomeren und Mischungen ihrer optischen Isomeren.

4. Verfahren nach Anspruch 1, zur Herstellung von Verbindungen der Formel I, worin $R_1$ Amino ist, $R_2$ Hydroxy ist, und $R_3$ Hydroxy bedeutet, ihrer pharmazeutisch verwendbaren Salze, ihrer optischen Isomeren und Mischungen ihrer optischen Isomeren.

5. Verfahren nach Anspruch 1 zur Herstellung von Verbindungen der Formel I, die in 5-Stellung die R-Konfiguration und in 6-Stellung die S-Konfiguration besitzen, und ihrer pharmazeutisch verwendbaren Salze.

6. Verfahren nach Anspruch 1 zur Herstellung von trans-2-(4-Aminobutyl)-6-hydroxymethyl-2-penem-3-carbonsäure und pharmazeutisch verwendbarer Salze davon.

7. Verfahren nach Anspruch 1 zur Herstellung von (5R,6S)-2-(4-Aminobutyl)-6-hydroxymethyl-2-penem-3-carbonsäure und pharmazeutisch verwendbarer Salze davon.

8. Verfahren zur Herstellung von pharmazeutischen Präparaten, dadurch gekennzeichnet, dass man eine nach dem Verfahren gemäss Anspruch 1 erhaltene Verbindung der Formel I mit einem pharmazeutisch verwendbaren Trägerstoff mischt.

0082113

Europäisches Patentamt

**EUROPÄISCHER TEILRECHERCHENBERICHT,**
der nach Regel 45 des Europäischen Patent-übereinkommens für das weitere Verfahren als europäischer Recherchenbericht gilt

Nummer der Anmeldung

EP 82 81 0511

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. [3]) |
|---|---|---|---|
| D,X | DE - A - 2 950 898 (BRISTOL-MYERS)<br>* Ansprüche 1,3,5,10,11; Seite 123, Beispiel 16, 8e Verbindung *<br>-- | 1-10, 12,13 | C 07 D 499/00<br>A 61 K 31/43//<br>C 07 D 515/04<br>C 07 D 205/08<br>C 07 F 9/65<br>C 07 F 7/18 |
| A | EP - A - 0 002 210 (MERCK)<br>* Ansprüche 1,4-6 *<br>----- | 1,8,12 | |
| | | | RECHERCHIERTE SACHGEBIETE (Int. Cl. [3])<br>C 07 D 499/00<br>A 61 K 31/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-10,12,13
Unvollständig recherchierte Patentansprüche:
Nicht recherchierte Patentansprüche: 11
Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| Den Haag | 09-02-1983 | CHOULY |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument